# EUROPEAN PATENT SPECIFICATION

(11) **EP 3 160 389 B1**
(45) Date of publication and mention of the grant of the patent: **17.04.2019**
(21) Application number: 15810986.8
(22) Date of filing: 18.06.2015
(51) Int. Cl.: A61F 2/04, C23C 28/00, C25D 7/04, C25D 11/02

(54) **NICKEL TITANIUM OXIDE COATED ARTICLES**
MIT NICKELTITANOXID BESCHICHTETE ARTIKEL
ARTICLES REVÊTUS D'OXYDE DE NICKEL ET DE TITANE

(30) Priority: 24.06.2014 US 201462016455 P
(43) Date of publication of application: 03.05.2017
(73) Proprietor: The Regents of the University of California, Oakland, CA 94607 (US)
(72) Inventor: DESAI, Tejal Ashwin, San Francisco, California 94118 (US); LEE, Phin Peng, Charleston, South Carolina 29407 (US)
(74) Representative: Williams Powell
(86) International application number: PCT/US2015/036503
(87) International publication number: WO 2015/200099

(56) References cited:
- WO-A1-2014/088944
- US-A1- 2008 086 195
- XIAOLING LI ET AL: "Nickel/Copper nanoparticles modified TiO2 nanotubes for non-enzymatic glucose biosensors", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 181, 1 May 2013 (2013-05-01), pages 501-508, XP055434130, NL ISSN: 0925-4005, DOI: 10.1016/j.snb.2013.02.035
- LOW C T J ET AL: "Electrodeposition and tribological characterisation of nickel nanocomposite coatings reinforced with nanotubular titanates", SURFACE AND COATINGS TECHNOLOGY, ELSEVIER BV, AMSTERDAM, NL, vol. 205, no. 7, 25 December 2010 (2010-12-25), pages 1856-1863, XP027533881, ISSN: 0257-8972 [retrieved on 2010-08-19]
- SHABALOVSKAYA, S ET AL.: 'Critical overview of Nitinol surfaces and their modifications for medical applications' ACTA BIOMATERIALIA vol. 4, no. 3, 06 February 2008, ISSN 1742-7061 pages 447 - 467, XP022588825
- HAMMOUDA, IM ET AL.: 'Micro-Photographic Analysis of Titanium Anodization to Assess Bio-Activation' JOURNAL OF DENTISTRY, MEDICINE AND MEDICAL SCIENCE vol. 3, no. 1, January 2014, pages 7 - 17, XP055248213

## Description

### TECHNICAL FIELD

This disclosure relates to an article comprising at least one substrate and at least one porous coating formed on the at least one substrate. This disclosure particularly relates to a medical article. This disclosure also relates to a porous coating comprising a compound of nickel, titanium and oxygen. This disclosure further relates to a porous coating comprising tubular structures.

### DESCRIPTION OF RELATED ART

Heart disease places an enormous burden on patients and is estimated to cost over $316 billion in the United States in 2010 alone, the majority of which is spent on coronary heart diseases. A substantial number of these patients who received percutaneous transluminal coronary angioplasty went on to suffer recurring ischemia. Within six months of the treatment, 30% to 60% of angioplasty patients suffer from restenosis. Bare metal stents (BMS) helped in the prevention of early abrupt closure due to elastic recoil and generally improved patient outcomes. However, in-stent restenosis (ISR), rates were still reported to be around 20% to 30%.

ISR after BMS implantation has been mainly attributed to neointimal hyperplasia, which is the result of vascular smooth muscle cells (VSMC), responding to the injury caused during stenting. These cells respond to the implantation by migrating into the vessel lumen, where they proliferate excessively and secrete an abundance of extracellular matrix (ECM) proteins, thereby re-narrowing the artery. The innovation of drug-eluting stents (DES) that release drugs that reduce the proliferation and migration of VSMC have successfully reduced ISR rates to below 10%. However, the anti-proliferative drugs in DES are non-specific and affect endothelial cells (EC) as well, resulting in delayed healing and an increase in the rates of late stent thrombosis. Specifically, drugs such as Paclitaxel and Rapamycin were known to increase the thrombogenic risk by reducing endothelial cell function, proliferation and migration.

Recent studies have shown that an upright nanotubular coating on titania substrates created via an anodization process has the potential to regulate EC and VSMC in a "pro-healing" manner. When cultured on these nanotube-coated substrates, VSMC have been shown to decrease in proliferation, motility and expression of genes related to inflammation, while EC have been shown to increase in proliferation, motility and secretion of prostaglandin - an anti-thrombogenic and anti-proliferative agent for VSMC.

Above summary of the related art is based on the following publications: Roger et al. "Heart Disease and Stroke Statistics. 2012 Update: A Report from the American Heart Association" Circulation 2012, 125, e2-e220; Rajagopal "Coronary Restenosis: A Review of Mechanisms and Management" Am. J. Med. 2003, 115, 547-553; Lüscher et al. "Drug-Eluting Stent and Coronary Thrombosis: Biological Mechanisms and Clinical Implications" Circulation 2007, 115, 1051-1058; Khan et al. "Drug Eluting Stents:
Developments and Current Status" J. Control. Release 2012; Mani et al. "Coronary Stents: A Materials Perspective. Biomaterials" 2007, 28, 1689-1710; Regan et al.
"Molecular Mechanisms of Decreased Smooth Muscle Differentiation Marker Expression after Vascular Injury" J. Clin. Invest. 2000, 106, 1139-1147; Joner et al.
"Pathology of Drug-Eluting Stents in Humans: Delayed Healing and Late Thrombotic Risk" J. Am. Coll. Cardiol. 2006, 48, 193-202; Finn et al. "Vascular Responses to Drug Eluting Stents: Importance of Delayed Healing. Arterioscler" Thromb. Vasc. Biol. 2007, 27, 1500-1510; Daemen et al. "Early and Late Coronary Stent Thrombosis of Sirolimus-Eluting and Paclitaxel-Eluting Stents in Routine Clinical Practice: Data from a Large Two-Institutional Cohort Study" Lancet 2007, 369, 667-678; Wenaweser et al.
"Incidence and Correlates of Drug-Eluting Stent Thrombosis in Routine Clinical Practice. 4-Year Results from a Large 2-lnstitutional Cohort Study" J. Am. Coll. Cardiol. 2008, 52, 1134-1140; McFadden et al. "Late Thrombosis in Drug-Eluting Coronary Stents after Discontinuation of Antiplatelet Therapy" Lancet 2004, 364, 1519-1521;
Steffel "Rapamycin, but Not FK-506, Increases Endothelial Tissue Factor Expression: Implications for Drug-Eluting Stent Design" Circulation 2005, 112, 2002-2011; Steffel et al. "Biological Effects of Drug-Eluting Stents in the Coronary Circulation" Herz 2007, 32, 268-273; Peng et al. "The Effect of TiO₂ Nanotubes on Endothelial Function and
Smooth Muscle Proliferation" Biomaterials 2009, 30, 1268-1272; Peng et al. "Whole Genome Expression Analysis Reveals Differential Effects of TiO2 Nanotubes on Vascular Cells" Nano Lett. 2010, 10, 143-148; Duerig et al. "An Overview of Nitinol Medical Applications" Mater. Sci. Eng. 1999, 275, 149-160; and Jin et al. "Composition Comprising Nanostructures for Cell, Tissue, and Artificial Organ Growth, and Methods for Making and Using Same" publication of a United States Patent, US 8,414,908.

Preparation of arrays of 38 nanometers (nm) outer diameter NiO-TiO₂ Nanotubes was disclosed in a publication by Kim et al. "Microstructure and Pseudocapacitive Properties of Electrodes Constructed of Oriented NiO-TiO2 Nanotube Arrays" Nano Lett. 2010, 10, 4099-4104. Synthesis and characterization of hybrid micro/nano-structured NiTi surfaces was disclosed in a publication by Huan et al. "Synthesis and Characterization of Hybrid Micro/Nano-structured NiTi Surfaces by a Combination of Etching and Anodizing" Nanotechnology, 2014, 25(5), 055602 (9 pages). Corrosion resistance and biocompatibility of NiTi shape memory alloys were disclosed in a publication by Pourmahdavi et al. "Effects of Anodic Oxidation in Ethylene Glycol Based Electrolyte on the Corrosion Resistance and Biocompatibility of NiTi Shape Memory Alloys" Adv. Mat. Res. 2014, 829, 431-435. WO 2014/088944 discloses: a titanium stent coated with an array of titania nanotubes.

### SUMMARY

This disclosure relates to an article which is a stent comprising:a substrate comprising at least one surface, and a coating comprising a compound of nickel, titanium and oxygen,wherein the coating is formed on the at least one surface of the substrate, and wherein the coating is a porous coating comprising an array of nanotubes, wherein the nanotubes have an average inner diameter of 70 nm or 110 nm.

The average inner diameter of the nanotube may be at most 95 % of the average outer diameter of the nanotube. The average inner diameter of the nanotube may also be at most 90 % of the average outer diameter of the nanotube. The average inner diameter of the nanotube may also be at most 80 % of the average outer diameter of the nanotube. The average lateral spacing of the nanotubes may be in the range of 1 nanometer to 1,000 nanometers. The average lateral spacing of the nanotubes may be in the range of 5 nanometers to 500 nanometers. The average lateral spacing of the nanotube may be in the range of 10 nanometers to 200 nanometers. The nanotubes may comprise a nanotube array of at least 1 nanotubes/micrometer². The nanotubes may also comprise a nanotube array of at least 10 nanotubes/micrometer². The nanotubes may also comprise a nanotube array of at least 100 nanotubes/micrometer². The nanotubes may also comprise a nanotube array of at least 1,000 nanotubes/micrometer².

The at least one nanotube may also comprise an oxide of nickel and an oxide of titanium. The at least one nanotube may also comprise a mixture of NiO and TiO₂.

An article may comprise at least one substrate and at least one coating wherein the substrate has at least one surface, and wherein the coating may be on the at least one surface of the substrate.

The substrate comprises a metal. The metal may comprise a stainless steel, an alloy of cobalt and chromium, an alloy of nickel and titanium, or mixtures thereof. The metal may also comprise an alloy of nickel and titanium. The metal may comprise Nitinol.

An article may comprise at least one substrate and at least one porous coating wherein the substrate may have at least one surface, wherein the coating may be on the at least one surface of the substrate, wherein the at least one substrate may comprise at least one metal, and wherein the at least one coating comprises a compound of nickel, titanium and oxygen,wherein the coating is formed on the at least one surface of the substrate, and wherein the coating is a porous coating comprising an array of nanotubes, wherein the nanotubes have an average inner diameter of 70 nm or 110 nm.

An article may comprise at least one substrate and at least one coating wherein the substrate may have at least one surface, wherein the coating may be on the at least one surface of the substrate, wherein the at least one substrate may comprise at least one metal comprising an alloy of nickel and titanium, and wherein the at least one coating comprises a compound of nickel, titanium and oxygen,wherein the coating is formed on the at least one surface of the substrate, and wherein the coating is a porous coating comprising an array of nanotubes, wherein the nanotubes have an average inner diameter of 70 nm or 110 nm.

An article may comprise at least one substrate and at least one coating wherein the substrate may have at least one surface, wherein the coating may be on the at least one surface of the substrate, wherein the at least one substrate may comprise at least one metal comprising an alloy of nickel and titanium, and wherein the at least one coating comprises a compound of nickel, titanium and oxygen,wherein the coating is formed on the at least one surface of the substrate, and wherein the coating is a porous coating comprising an array of nanotubes, wherein the nanotubes have an average inner diameter of 70 nm or 110 nm.

The article or substrate comprises a stent.

A method of preparation of an article may comprise: providing at least one substrate, which is a stent, wherein the at least one substrate comprises at least one metal comprising an alloy of nickel and titanium, wherein the at least one metal may be at least 0.1 weight percent of the at least one substrate, and wherein the at least one substrate has at least one surface; forming at least one anode comprising the at least one substrate; providing at least one cathode; submerging at least a portion of the at least one anode and at least a portion of the at least one cathode in an electrolyte solution; applying electrical energy between the at least one anode and the at least one cathode for a period sufficient to form a coating on at least one surface of the at least one substrate, wherein the coating comprises a compound of nickel, titanium and oxygen,wherein the coating is formed on the at least one surface of the substrate, and wherein the coating is a porous coating comprising an array of nanotubes, wherein the nanotubes have an average inner diameter of 70 nm or 110 nm, and thereby preparing the article.

The method may comprise reducing oxygen level at and/or close to the substrate surface below a detection limit of an energy dispersive X-ray spectrometer, wherein the reducing the amount of oxide layer is carried out before the step of providing at least one substrate.

The method may comprise testing the thereby formed article to determine number and/or morphology of primary human aortic endothelial cells migrated to, spread, or proliferated on the surface of the article in vitro, and thereby obtaining a test result for re-endothelialization potential of the article.

The method may comprise testing the substrate prior to the step of the providing at least one substrate, wherein the testing may comprise determining number and/or morphology of primary human aortic endothelial cells migrated to, spread, or proliferated on the surface of the substrate in vitro, and thereby obtaining a test result for re-endothelialization potential of the substrate.

The method may comprise comparing the test result for the re-endothelialization potential of the article with the test result for the re-endothelialization potential of the substrate, and deciding whether to use the thereby formed article in manufacturing of a medical device.

The method may comprise comparing the test result for the re-endothelialization potential of the article with the test result for the re-endothelialization potential of the substrate, and using the thereby formed article in manufacturing of a medical device if number of migrated primary human aortic endothelial cells for the article may be higher than that for the substrate.

The method may comprise comparing the test result for the re-endothelialization potential of the article with the test result for the re-endothelialization potential of the substrate, and using the thereby formed article in manufacturing of a medical device if number of migrated primary human aortic endothelial cells for the article may be at least 10% higher than that for the substrate.

The method may comprise comparing the test result for the re-endothelialization potential of the article with the test result for the re-endothelialization potential of the substrate, and using the thereby formed article in manufacturing of a medical device if number of migrated primary human aortic endothelial cells for the article may be at least 50% higher than that for the substrate.

The method may comprise comparing the test result for the re-endothelialization potential of the article with the test result for the re-endothelialization potential of the substrate, and using the thereby formed article in manufacturing of a medical device if number of migrated primary human aortic endothelial cells for the article is at least 100% higher than that for the substrate.

The method may comprise testing the thereby formed article to determine number and/or morphology of human aortic smooth muscle cells migrated to, spread, or proliferated on the surface of the article in vitro, and thereby obtaining a test result for restenosis reduction potential of the article.

The method may comprise testing the substrate prior to the step of the providing at least one substrate, wherein the testing comprises determining number and/or morphology of primary human aortic smooth muscle cells migrated to, spread, or proliferated on the surface of the substrate in vitro, and thereby obtaining a test result for restenosis reduction potential of the substrate.

The method may comprise comparing the test result for the restenosis reduction potential of the article with the test result for the restenosis reduction potential of the substrate, and deciding whether to use the thereby formed article in manufacturing of a medical device.

The method may comprise comparing the test result for the restenosis reduction potential of the article with the test result for the restenosis reduction potential of the substrate, and using the thereby formed article in manufacturing of a medical device if number of migrated human aortic smooth muscle cells for the article may be lower than that for the substrate.

The method may comprise comparing the test result for the restenosis reduction potential of the article with the test result for the restenosis reduction potential of the substrate, and using the thereby formed article in manufacturing of a medical device if number of migrated human aortic smooth muscle cells for the article may be at least 10% lower than that for the substrate.

The method may comprise comparing the test result for the restenosis reduction potential of the article with the test result for the restenosis reduction potential of the substrate, and using the thereby formed article in manufacturing of a medical device if number of migrated human aortic smooth muscle cells for the article may be at least 50% lower than that for the substrate.

The step of applying electrical energy between the at least one anode and the at least one cathode may comprise applying a substantially constant voltage. The substantially constant voltage may be in the range of 1 volt to 1,000 volts. The substantially constant voltage may be in the range of 10 volt to 100 volts. The substantially constant voltage may be about 85 volts. The substantially constant voltage may be about 70 volts.

The step of applying electrical energy between the at least one anode and the at least one cathode may comprise applying a substantially constant current. The substantially constant current may be in the range of 1 femtoampere to 100 kiloamperes.

The period may be in the range of 0.1 minute to 1,000 minutes. The period may be in the range of 1 minute to 100 minutes. The period may be about 4 minutes.

The electrolyte solution may comprise compounds of ammonium fluoride, ethylene glycol, and water.

The method may comprise maintaining the electrolyte solution at a substantially constant temperature. The substantially constant temperature may be above a freezing point of the electrolyte solution and below a boiling point of the electrolyte solution. The substantially constant temperature may be in the range of 10 °C to 50 °C.

### BRIEF DESCRIPTION OF DRAWINGS

The drawings disclose illustrative embodiments. They do not set forth all embodiments. Other embodiments may be used in addition or instead. Details that may be apparent or unnecessary may be omitted to save space or for more effective illustration. Conversely, some embodiments may be practiced without all of the details that are disclosed. When the same numeral appears in different drawings, it is intended to refer to the same or like components or steps.

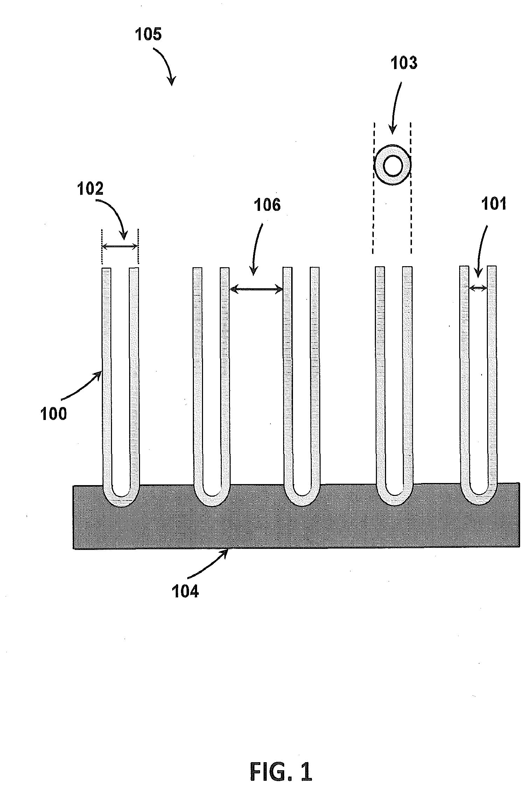

**FIG. 1****:** A drawing schematically showing a cross-sectional view of a nanotubular array on a substrate.

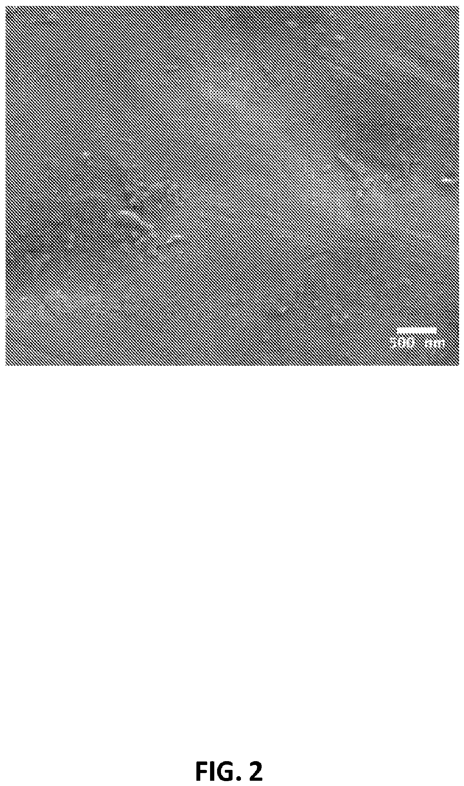

**FIG. 2****:** A scanning electron microscope (SEM) image of an exemplary substrate. The substrate is Nitinol foil purchased from Alfa Aesar. The white scale bar on the image is about 500 nanometers (nm).

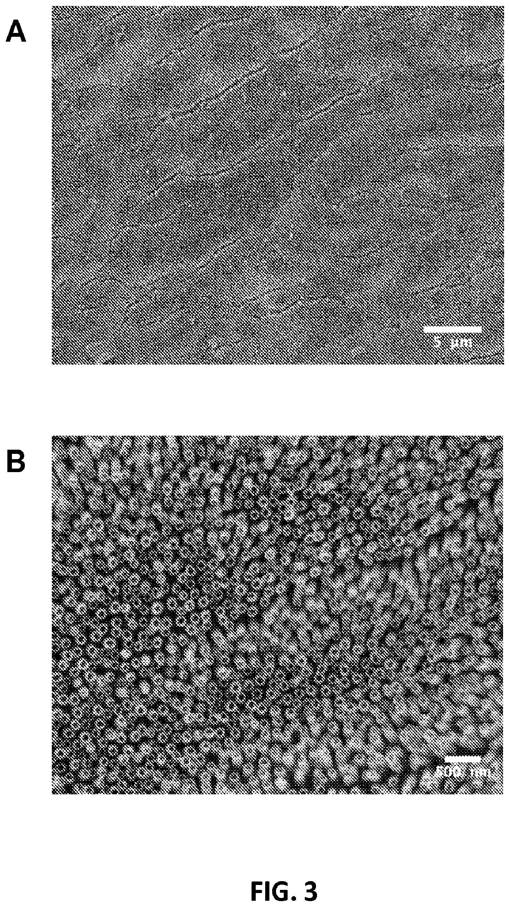

**FIG. 3****:** An SEM image of an exemplary nickel-titanium-oxide coating formed on a Nitinol foil. The white scale bars on the image are **(A)** about 5 micrometers (µm) and **(B)** about 500 nm. The Nitinol foil was purchased from Alfa Aesar.

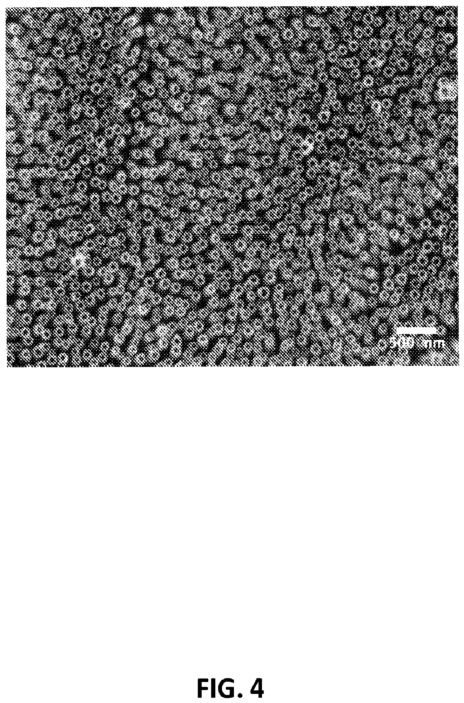

**FIG. 4****:** An SEM image of an exemplary nickel-titanium-oxide coating formed on a Nitinol foil. The white scale bar on the image is about 500 nm. The Nitinol foil was purchased from Alfa Aesar.

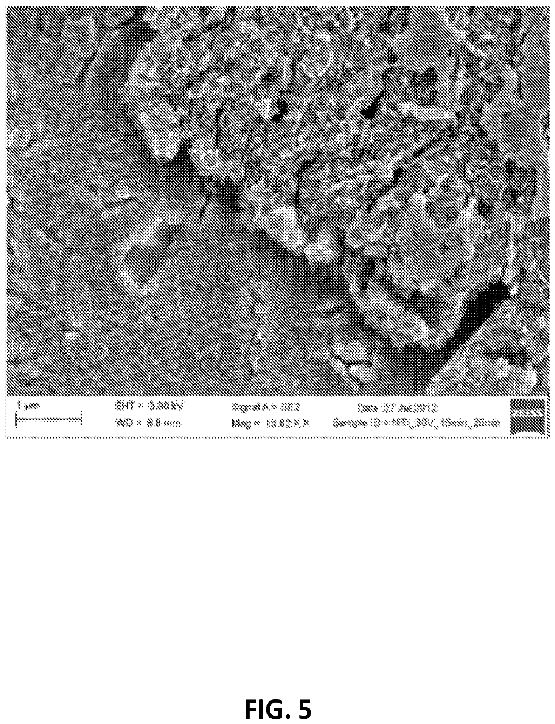

**FIG. 5****:** An SEM image of an exemplary nickel-titanium-oxide coating formed on a Nitinol foil. The scale bar below the image is about 1 µm. The Nitinol foil was purchased from NDC. The electrolysis was carried out at about 30 volts (V) for about 15 minutes (min). The electrolyte comprised about 1.48 grams (g) of NH₄F, about 490 milliliters (ml) of ethylene glycol, and about 8.35 ml of water.

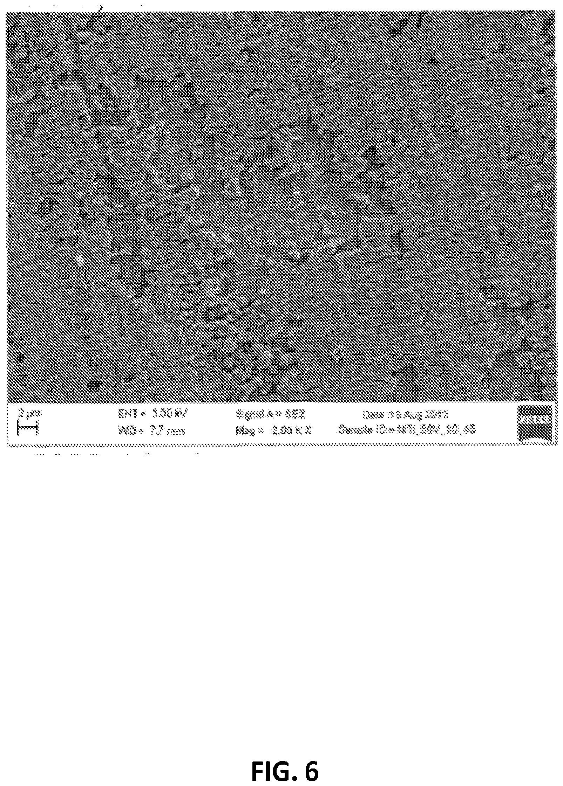

**FIG. 6****:** An SEM image of an exemplary nickel-titanium-oxide coating formed on a Nitinol foil. The scale bar below the image is about 2 µm. The Nitinol foil was purchased from NDC. The electrolysis was carried out at about 50 V for about 10 min. The electrolyte comprised about 1.48 g of NH₄F, about 490 ml ethylene glycol, and about 8.35 ml water.

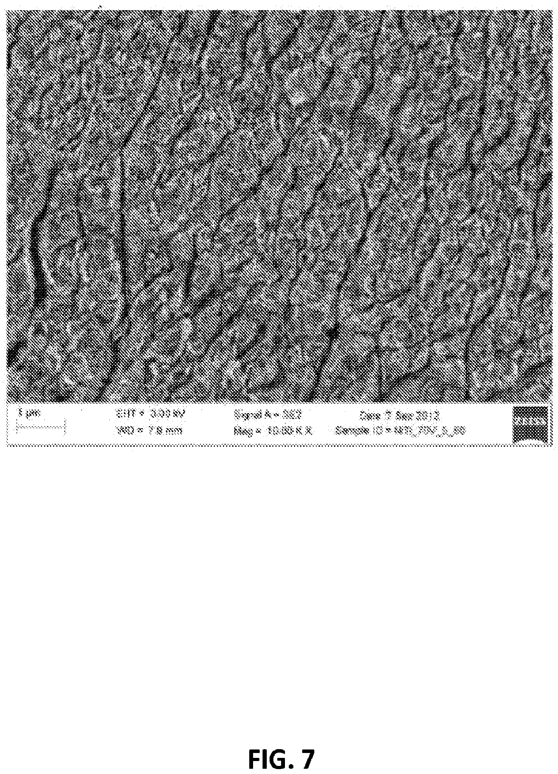

**FIG. 7****:** An SEM image of an exemplary nickel-titanium-oxide coating formed on a Nitinol foil. The scale bar below the image is about 1 µm. The Nitinol foil was purchased from NDC. The electrolysis was carried out at about 70 V for about 5 min. The electrolyte comprised about 1.48 g NH₄F, about 490 ml ethylene glycol, and about 8.35 ml water.

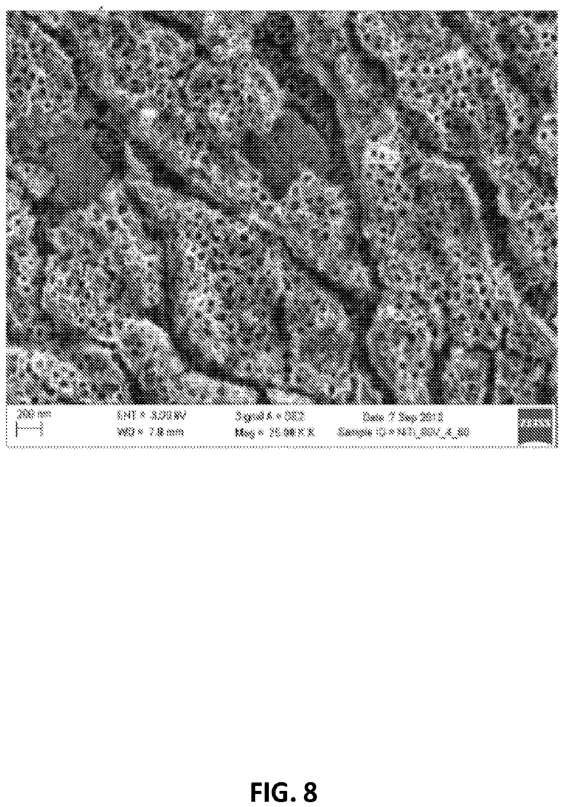

**FIG. 8****:** An SEM image of an exemplary nickel-titanium-oxide coating formed on a Nitinol foil. The scale bar below the image is about 200 nm. The Nitinol foil was purchased from NDC. The electrolysis was carried out at about 80 V for about 4 min. The electrolyte comprised about 1.48 g NH₄F, about 490 ml ethylene glycol, and about 8.35 ml water.

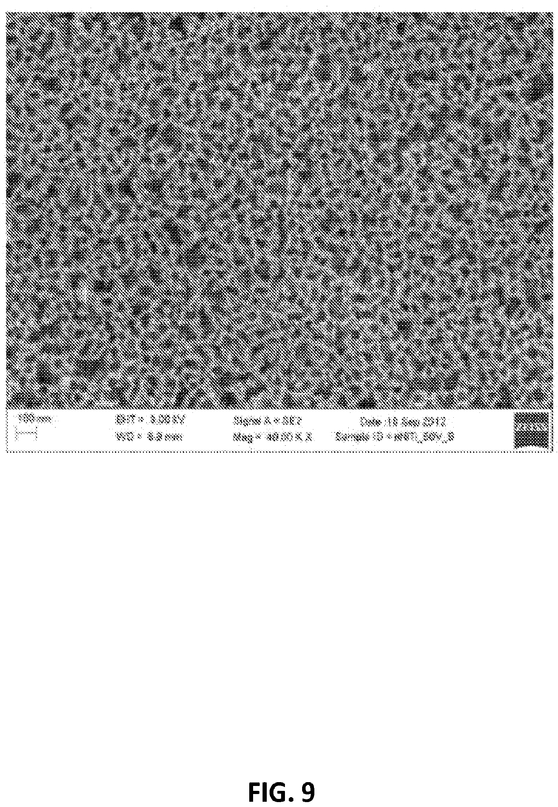

**FIG. 9****:** An SEM image of an exemplary nickel-titanium-oxide coating formed on a Nitinol foil. The scale bar below the image is about 100 nm. The Nitinol foil was purchased from Alfa Aesar. The electrolysis was carried out at about 50 V for about 9 min. The electrolyte comprised about 1.50 g NH₄F, about 490 ml ethylene glycol, and about 10 ml water.

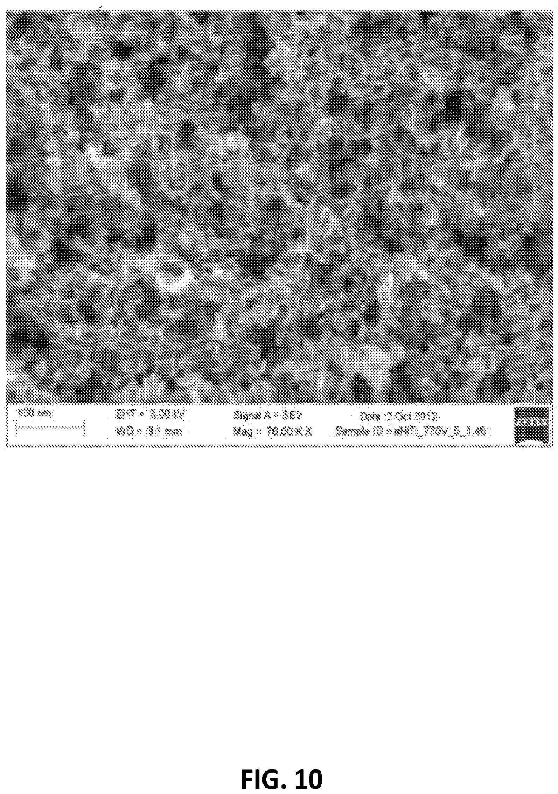

**FIG. 10****:** An SEM image of an exemplary nickel-titanium-oxide coating formed on a Nitinol foil. The scale bar below the image is about 100 nm. The Nitinol foil was purchased from Alfa Aesar. The electrolysis was carried out at about 70 V for about 5 min. The electrolyte comprised about 1.45 g NH₄F, about 490 ml ethylene glycol, and about 10 ml water

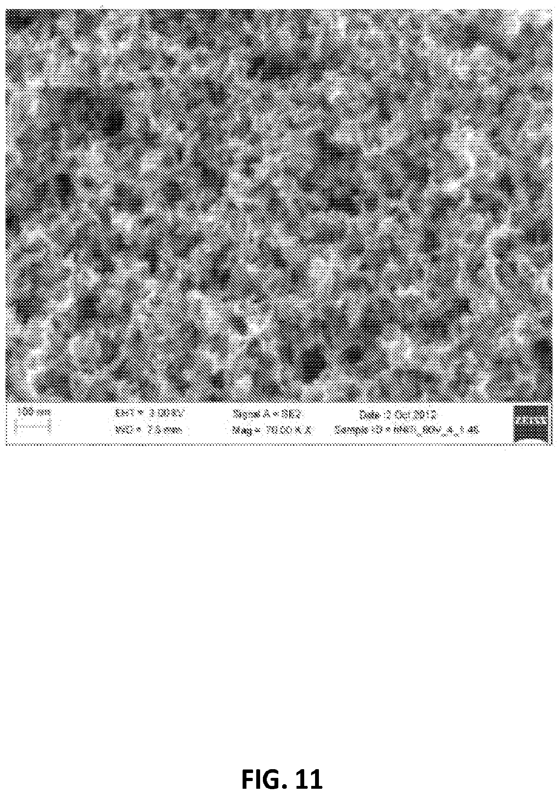

**FIG. 11****:** An SEM image of an exemplary nickel-titanium-oxide coating formed on a Nitinol foil. The scale bar below the image is about 100 nm. The Nitinol foil was purchased from Alfa Aesar. The electrolysis was carried out at about 80 V for about 4 min. The electrolyte comprised about 1.45 g NH₄F, about 490 ml ethylene glycol, and about 10 ml water.

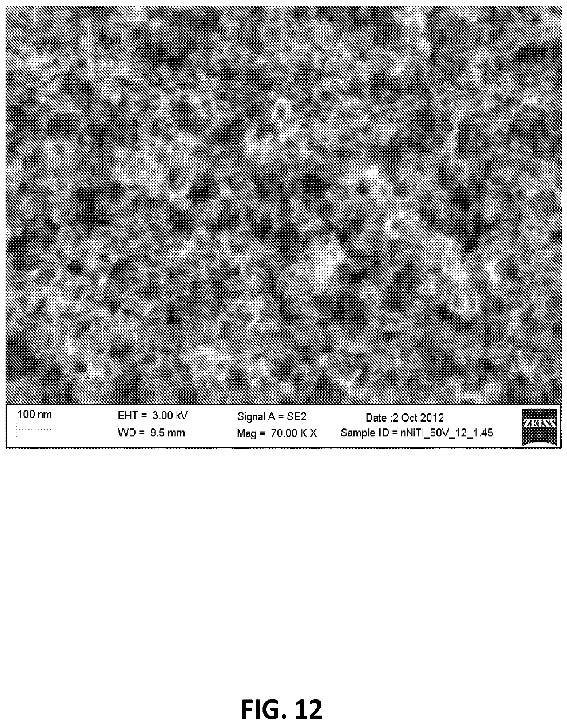

**FIG. 12****:** An SEM image of an exemplary nickel-titanium-oxide coating formed on a Nitinol foil. The scale bar below the image is about 100 nm. The Nitinol foil was purchased from Alfa Aesar. The electrolysis was carried out at about 50 V for about 12 min. The electrolyte comprised about 1.45 g NH₄F, about 490 ml ethylene glycol, and about 10 ml water.

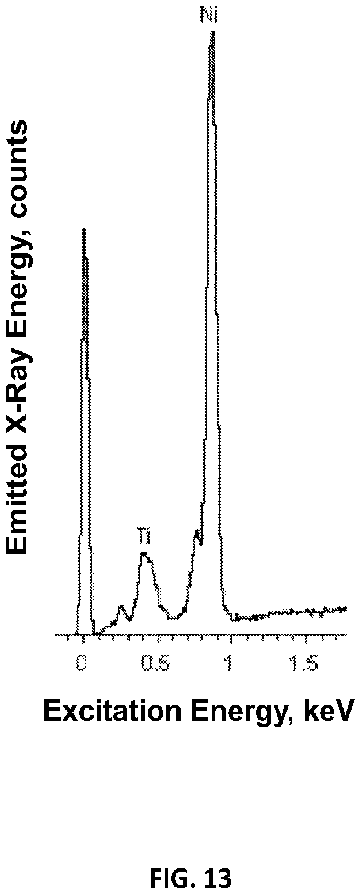

**FIG. 13****:** An energy dispersive X-Ray spectroscopy (EDS) of a surface of an exemplary Nitinol foil purchased from Alfa Aesar. The SEM image of this foil is shown in **FIG.2****.**

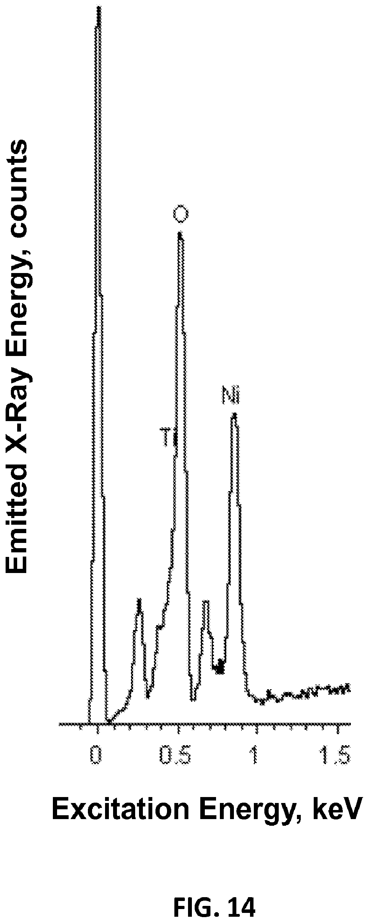

**FIG. 14****:** An EDS of a surface of an exemplary nickel-titanium-oxide coating formed on the bare Nitinol foil purchased from Alfa Aesar. The SEM image of this coating is shown in **FIG.3****.**

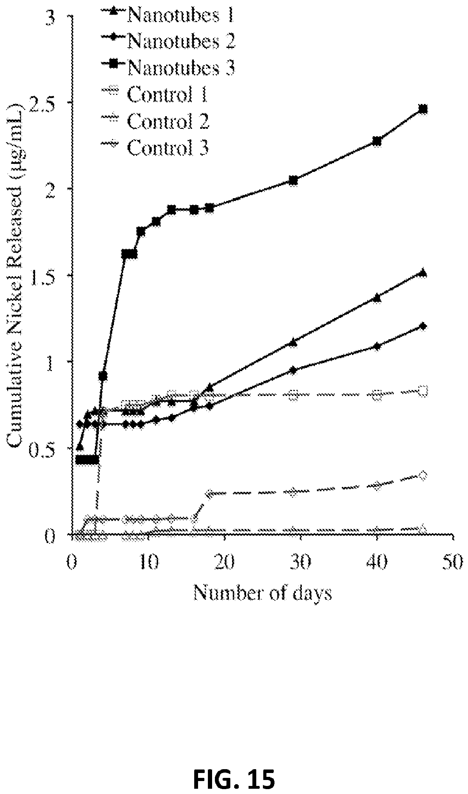

**FIG. 15****:** Cumulative nickel ions released from three samples each of the bare Nitinol foil (i.e. control 1, 2 and 3) purchased from Alfa Aesar, and the article comprising nanotubular structures (i.e. nanotubes 1, 2 and 3) shown in **FIG.3****.**

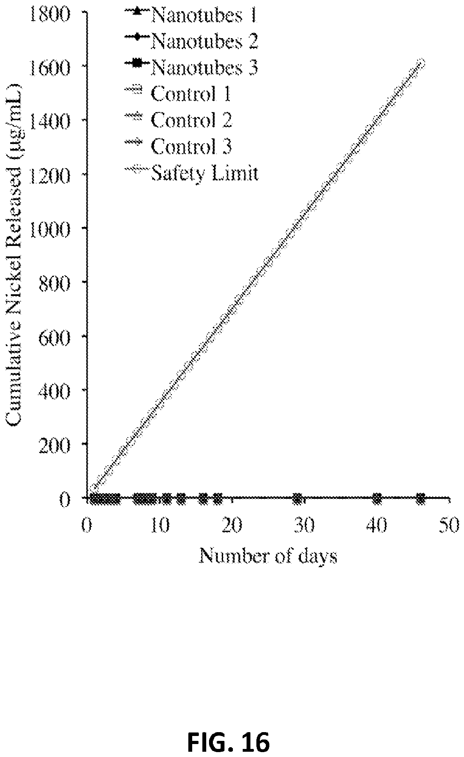

**FIG. 16****:** The safety limit of intravenous nickel contamination and the cumulative nickel ion released from three samples each of the bare Nitinol foil (i.e. control 1, 2 and 3) purchased from Alfa Aesar, and the article comprising nanotubular structures (i.e. nanotubes 1, 2 and 3). The cumulative nickel ions released from the article and the control were shown in **FIG.15** in detail.

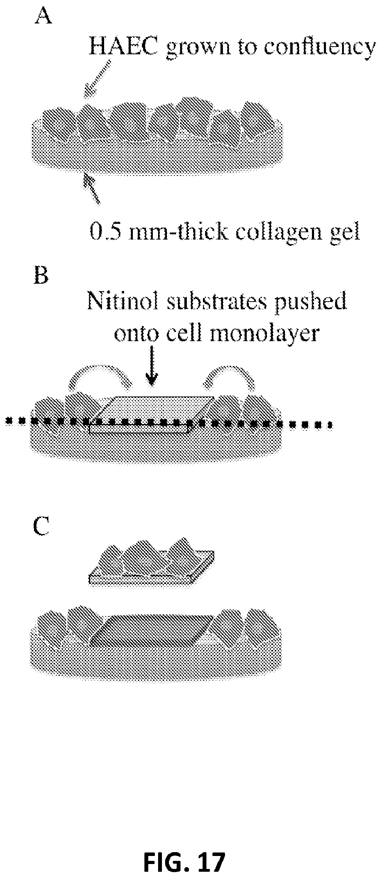

**FIG. 17****:** A drawing schematically describing the exemplary method used to determine number of human aortic endothelial cells (HAEC) migrated on surfaces of the bare Nitinol foil (i.e. control), and the article comprising nickel-titanium-oxide coating.

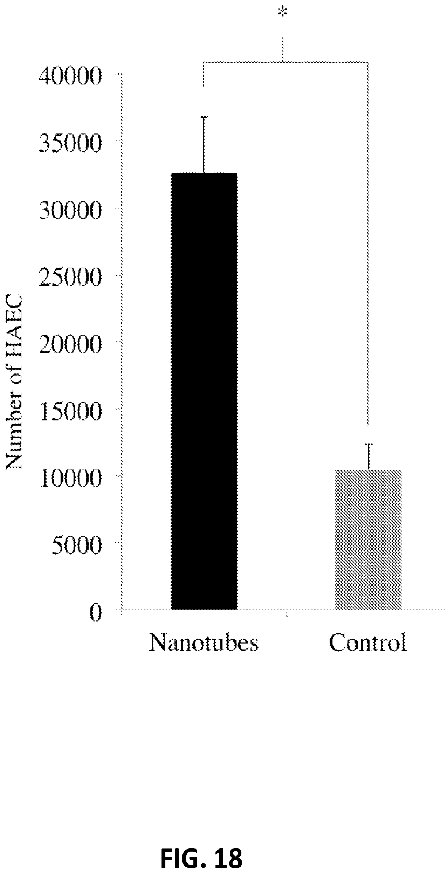

**FIG. 18****:** Number of HAEC migrated on the bare Nitinol foil (i.e. control), and the article comprising a nickel-titanium-oxide coating (i.e. nanotubes). * N = 5, p < 0.001 for each bar on the graph.

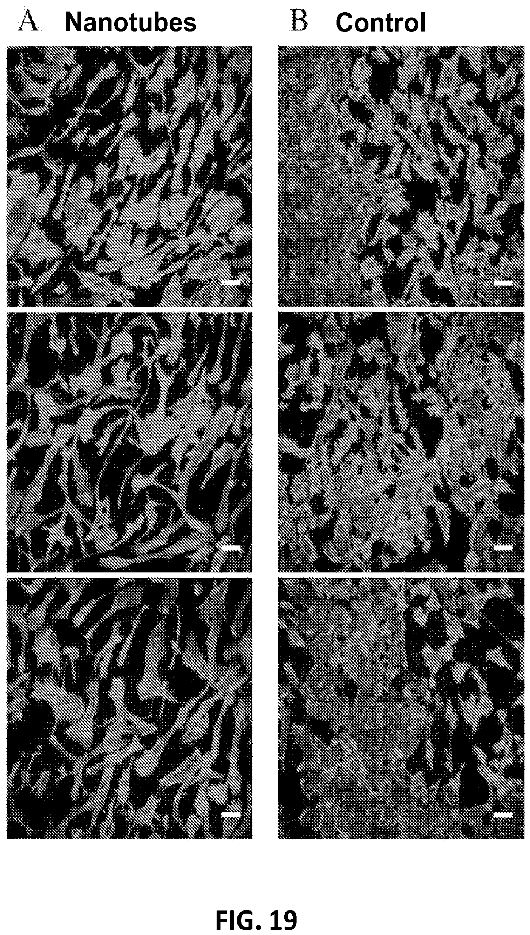

**FIG. 19****:** Fluorescence microscopy images of HAEC **(A)** on the surface of the article comprising a titanium-nickel-oxide coating (i.e. nanotubes), and **(B)** on the surface of the bare Nitinol foil (i.e. control) after about 7 days of culture. Three images for each sample were recorded to have qualitative analysis of the cell morphology. FITC-Phalloidin staining of F-actin is shown in red while DAPI staining of cell nuclei is shown in blue. White scale bars are about 50µm.

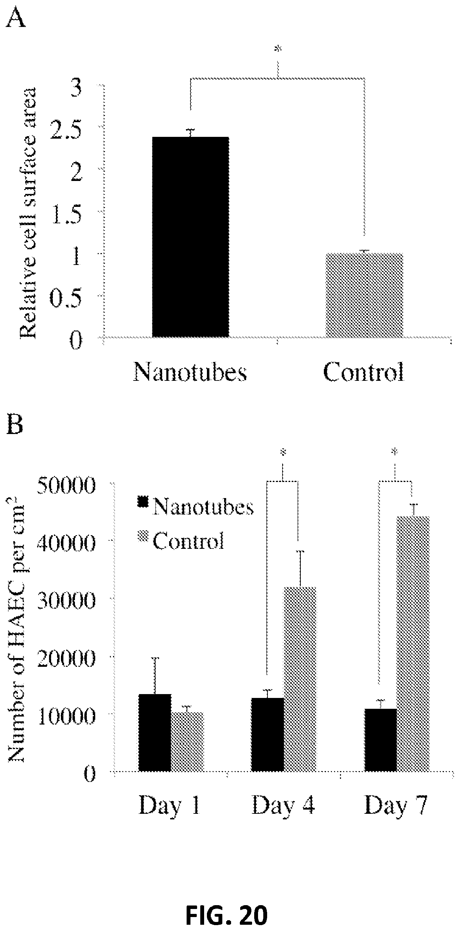

**FIG. 20****:** HAEC on the surface of the article comprising a titanium-nickel-oxide coating (i.e. nanotubes), and on the surface of bare Nitinol foil (i.e. control): **(A)** HAEC spreading is represented by the average cell surface area normalized to that of the control after about 7 days, and **(B)** HAEC growth is represented in number of HAEC per cm² after about 1 day, about 4 days, and about 7 days of culture. *N = 5, p < 0.01 for each bar on the graph.

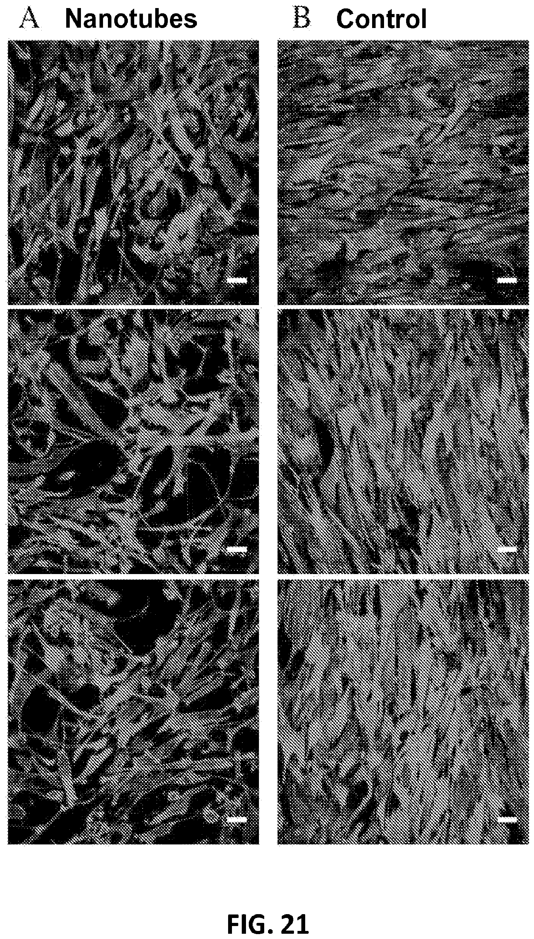

**FIG. 21****:** Fluorescence microscopy images of human aortic smooth muscle cells (HASMC) **(A)** on the surface of the article comprising a nickel-titanium-oxide coating (i.e. nanotubes), and **(B)** on the surface of the bare Nitinol foil (i.e. control) after about 7 days of culture. Three images for each sample were recorded to have qualitative analysis of the cell morphology. FITC-Phalloidin staining of F-actin is shown in red while DAPI staining of cell nuclei is shown in blue. White scale bars are about 50µm.

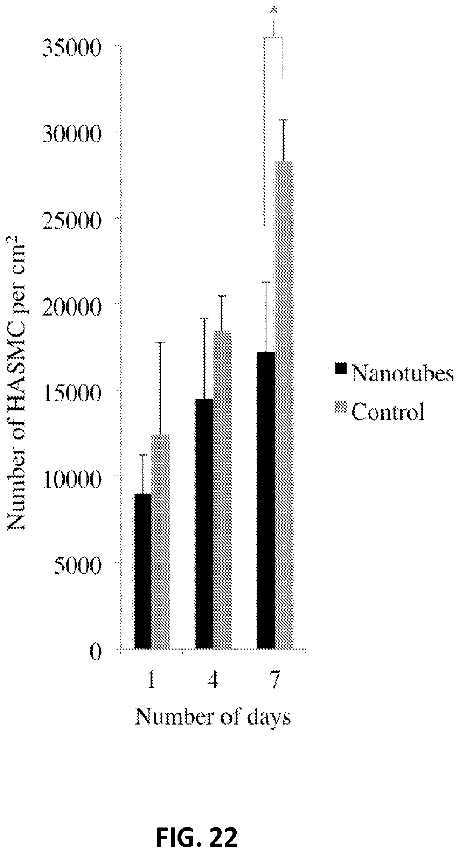

**FIG. 22****:** HASMC on the surface of the article comprising a nickel-titanium-oxide coating (i.e. nanotubes), and on the surface of the bare Nitinol foil (i.e. control): HASMC growth is represented in number of HASMC per cm² after about 1 day, about 4 days, and about 7 days of culture. *N = 5, p < 0.01 for each bar on the graph.

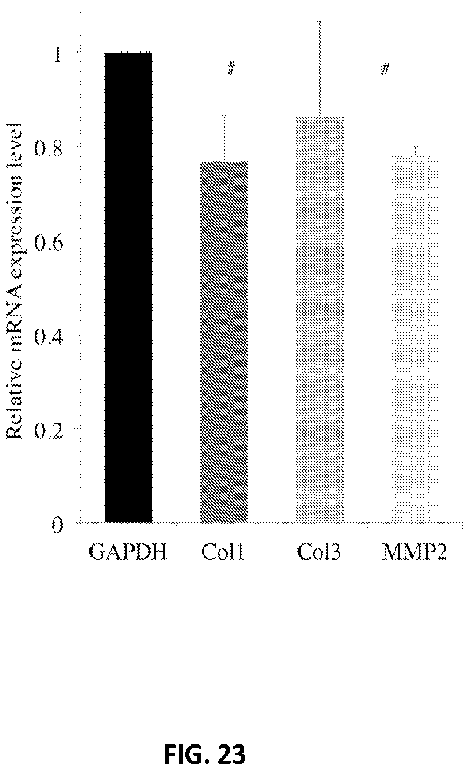

**FIG. 23****:** Effects of the nickel-titanium-oxide coating on the mRNA expression levels of Collagen 1 (Col1), Collagen 3 (Col3) and Matrix Metalloproteinase 2 (MMP2) genes in HASMC, relative to the housekeeping gene GAPDH. The relative mRNA expression levels of Col 1 and MMP 2 decreased significantly when cultured on the article comprising a titanium-nickel-oxide coating (i.e. nanotubes) as compared to the bare Nitinol foil (i.e. control). # N = 5, p < 0.05 for each bar on the graph.

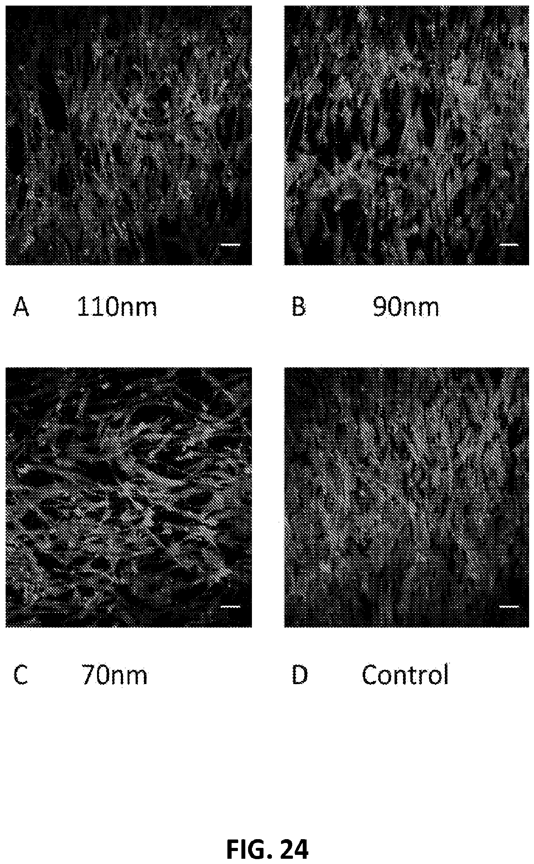

**FIG. 24****:** Fluorescence microscopy images of HASMC: on the surface of the articles comprising a titanium-nickel-oxide coatings (i.e. nanotubes): **(A)** about 110 nm inner diameter nanotubes, **(B)** about 90 nm inner diameter nanotubes, and **(C)** about 70 nm inner diameter tubes; and **(D)** on the surface of the bare Nitinol foil (i.e. control) after about 1 day of culture.

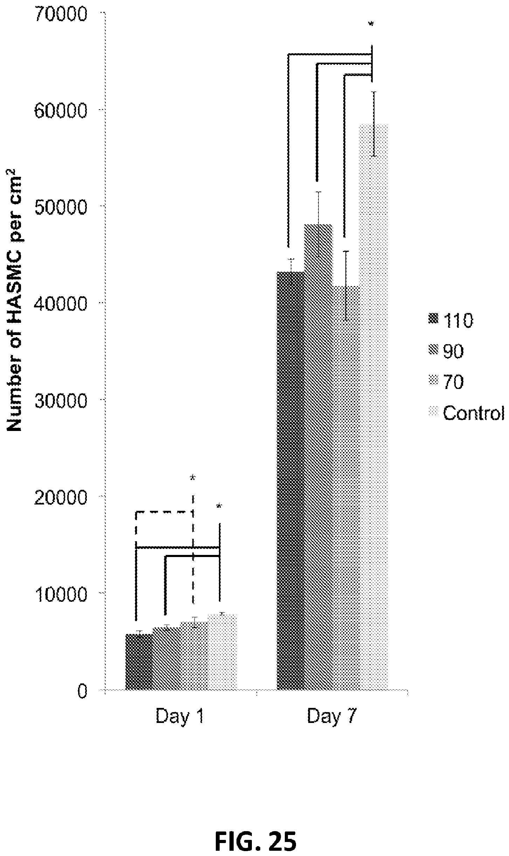

**FIG. 25****:** HASMC on the surface of the article comprising a nickel-titanium-oxide coating comprising about 110 nm inner diameter nanotubes ("110"), about 90 nm inner diameter nanotubes ("90"), about 70 nm inner diameter tubes ("70"); and on the surface of the bare Nitinol foil (i.e. control). HASMC adhesion and growth is represented in number of HASMC per cm² after about 1 day and 7 days of culture. *N = 5, p < 0.05 for each bar on the graph.

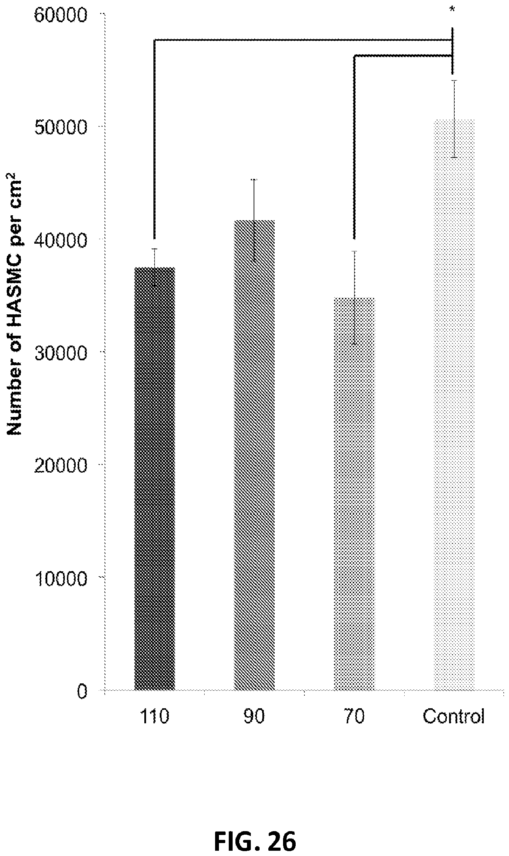

**FIG. 26****:** Difference in the HASMC number between after about 1 day and 7 days of culture. HASMC on the surface of the article comprising a nickel-titanium-oxide coating comprising about 110 nm inner diameter nanotubes ("110"), about 90 nm inner diameter nanotubes ("90"), and about 70 nm inner diameter tubes ("70"); and on the surface of the bare Nitinol foil (i.e. control). HASMC growth is represented in number of HASMC per cm². *N = 5, p < 0.05 for each bar on the graph.

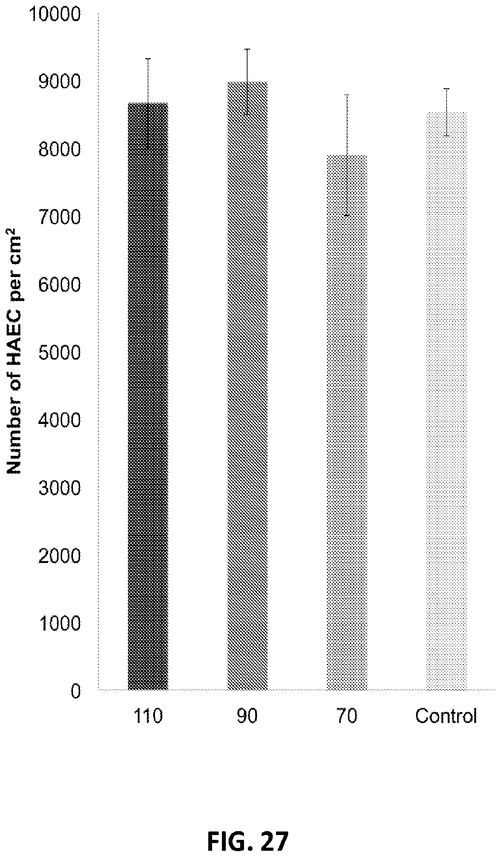

**FIG. 27****:** HAEC on the surface of the article comprising a nickel-titanium-oxide coating comprising about 110 nm inner diameter nanotubes ("110"), about 90 nm inner diameter nanotubes ("90"), and about 70 nm inner diameter tubes ("70"); and on the surface of the bare Nitinol foil (i.e. control). HAEC adhesion is represented in number of HAEC per cm² after about 1 day of culture. *N = 5, p < 0.05 for each bar on the graph.

### Detailed Description of Illustrative Embodiments

### Reference is made to:

Lee et al. "Nitinol-Based Nanotubular Coatings for the Modulation of Human Vascular Cell Function" Nano Lett., 2014, 14 (9), pp 5021-5028.

The compound of nickel-titanium-oxide may comprise any compound comprising nickel, titanium and oxygen. For example, the compound of nickel-titanium-oxide may comprise any mixture of an oxide of nickel and oxide of titanium. An oxide of nickel may be any oxide of nickel, for example, NiO, Ni₂O₃, NiO₂, or mixtures thereof. An oxide of titanium may be any oxide of titanium, for example, TiO, TiO₂, Ti₂O₃, or mixtures thereof. The compound of nickel-titanium-oxide may also comprise NiO-TiO₂.

The porous coating comprises at least one nanotube. Thus, the porous coating may comprise an array of nanotubes. Such porous coatings comprising nanotubular structures are hereafter referred to as "nanotubular" coatings. An example of such coating comprising tubes formed on a substrate is schematically shown in **FIG. 1****.** The plane of the horizontal cross section (103) of a nanotube may be parallel to the horizontal surface of the substrate (104).

The nanotube may not need to be a perfect hollow cylinder having perfectly circular horizontal cross section. The nanotube horizontal cross section may be non-circular. For example, it may be oval. Or, it may have irregular horizontal cross sectional shape.

The nanotube (100) has an inner diameter (101) and an outer diameter (102). The outer or inner diameter of the nanotube maybe measured, for example, by using a microscopic technique. For example, for a nanotube that has an elliptical cross section, the major axis of the ellipse may be the inner diameter of this nanotube. Or, in another example, for a nanotube that has a circular cross section, the diameter of the circle may be the inner diameter of this nanotube. The outer diameter or inner diameter of the nanotube may also be defined by measuring the maximum Feret diameter (i.e. the maximum caliper diameter). The Feret diameter may be defined as the distance between two parallel tangential lines restricting the cross sectional shape of an object. For example, for tubes that have irregular horizontal cross sections, the maximum Feret diameter may be used to define the inner diameter of the nanotube. The outer diameters of the nanotubes may similarly be defined.

The average nanotube outer diameter may be about 110 nm. The average nanotube outer diameter may be about 90 nm. The average inner diameter of the nanotube may be at most 95 % of the average nanotube outer diameter. The average nanotube inner diameter may be at most 90 % of the average nanotube outer diameter. The average nanotube inner diameter may be at most 80 % of the average nanotube outer diameter.

It may be easier for tissue cells, like endothelial cells, to grab surface of the article comprising discreet nanotubes as compared to that of the articles comprising nanotubes substantially touching each other. That is, migration, spreading and proliferation potential of tissue cells on the surface of the article comprising nanotube arrays (105) with a lateral spacing (106) between the tubes may be higher than that of the article with no lateral spacing between tubes. The lateral spacing between the nanotubes is the distance between the outer surfaces of the nanotubes. The average lateral nanotube spacing may be in the range of 1 nm to 1,000 nm. The average lateral nanotube spacing may also be in the range of 5 nm to 500 nm. The average nanotube lateral spacing may also be in the range of 10 nm to 200 nm. The average nanotube lateral spacing may be 85 nm. The average distance between tubes may be 85 nanometers. The average distance between tubes may be 63 nanometers.

In certain embodiments the nanotubes are fabricated such that they are in substantially orthogonal relationship to the surface of the substrate. For example, the angle between a lateral side of the nanotube and the surface of the substrate will be about 75° to about 110°, including about 80°, 85°, 90°, 95°, 100°, and 105°. As such, the nanotubes are generally in a vertical orientation with respect to the surface of the substrate.

An array of nanotubes may comprise at least 1 nanotubes/micrometer² (nanotubes/µm²), at least 10 nanotubes/µm², at least 100 nanotubes/µm², or at least 1,000 nanotubes/µm².

The at least one nanotube comprises a compound of nickel, titanium and oxygen (or "nickel-titanium-oxide"). The nickel-titanium-oxide compound may comprise any compound comprising nickel, titanium and oxygen. For example, the compound of nickel-titanium-oxide may comprise any mixture of an oxide of nickel and an oxide of titanium. The oxide of nickel may be any oxide of nickel, for example, NiO, Ni₂O₃, NiO₂, or mixtures thereof. The oxide of titanium may be any oxide of titanium, for example, TiO, TiO₂, Ti₂O₃, or mixtures thereof. The nickel-titanium-oxide compound may also comprise a mixture of NiO-TiO₂.

The stent comprises at least one substrate. The substrate has at least one surface. The coating is (formed) on the at least one surface of the substrate. The substrate may comprise a metal. The metal may comprise a stainless steel, an alloy of cobalt and chromium, an alloy of nickel and titanium, or mixtures thereof. The metal may also comprise an alloy of nickel and titanium. The metal may also comprise nitinol.

The stent may be any stent that is suitable in treatment of any blood vessels that have health problems. For example, the stent may be used to open clogged heart arteries.

The substrate may comprise a metal. The metal may comprise a stainless steel, an alloy of cobalt and chromium, an alloy of nickel and titanium, or mixtures thereof. The metal may also comprise an alloy of nickel and titanium. The metal may also comprise nitinol.

The stent according to claim 1 may have advantages over a bare metal stent. For example, the stent, when it is implanted in a blood vessel, may provide conditions that increase re-endothelialization and/or reduce restenosis as compared to the bare metal stent. Thereby, the wound caused by the implantation of the stent may heal better and faster when the article comprising a medical device and a porous coating, disclosed in this invention, is used for the treatment.

For example, the stent comprises at least one substrate and at least one porous coating wherein the substrate has at least one surface, wherein the coating is on the at least one surface of the substrate, wherein the at least one substrate may comprise at least one metal, and wherein the at least one coating comprises a compound of nickel, titanium and oxygen. In another example, the stent comprises at least one substrate and at least one coating wherein the substrate has at least one surface, wherein the coating is on the at least one surface of the substrate, wherein the at least one substrate may comprise at least one metal comprising an alloy of nickel and titanium, and wherein the at least one coating comprises a compound of nickel, titanium and oxygen. Yet in another example, the stent comprises at least one substrate and at least one coating wherein the substrate has at least one surface, wherein the coating is on the at least one surface of the substrate, wherein the at least one substrate may comprise at least one metal comprising an alloy of nickel and titanium, wherein the at least one coating comprises at least one nanotube comprising a compound of nickel, titanium and oxygen.

The stent may be prepared by any suitable method. For example, the article may be prepared by using an electrolysis method. The method of preparation of the stent may comprise providing at least one substrate, wherein the at least one substrate may have at least one surface; forming at least one anode comprising the at least one substrate; providing at least one cathode; submerging at least a portion of the at least one anode and at least a portion of the at least one cathode in an electrolyte solution; applying electrical energy between the at least one anode and the at least one cathode for a period sufficient to form a coating on at least one surface of the at least one substrate; and thereby preparing the stent.

In this method, the at least one substrate may comprise at least one metal comprising an alloy of nickel and titanium, wherein the at least one metal is at least 0.1 weight percent of the at least one substrate. The metal may comprise a stainless steel, an alloy of cobalt and chromium, an alloy of nickel and titanium, or mixtures thereof. The metal may also comprise an alloy of nickel and titanium. The metal may also comprise nitinol.

In this method, the coating may comprise an oxygen level that can be detected by an energy dispersive X-ray spectrometer. The coating comprises a compound of nickel, titanium and oxygen (or "nickel-titanium-oxide"). The compound of nickel-titanium-oxide may comprise any compound comprising nickel, titanium and oxygen. For example, the compound of nickel-titanium-oxide may comprise any mixture of an oxide of nickel and oxide of titanium. An oxide of nickel may be any oxide of nickel, for example, NiO, Ni₂O₃, NiO₂, or mixtures thereof. An oxide of titanium may be any oxide of titanium, for example, TiO, TiO₂, Ti₂O₃, or mixtures thereof. The compound of nickel-titanium-oxide may also comprise NiO-TiO₂.

In this method, the coating is a porous coating. The porous coating comprises at least one nanotube. Thus, the porous coating may comprise an array of the nanotubes. The average nanotube outer diameter may be about 110 nanometers. The average nanotube outer diameter may be about 90 nanometers. The average inner diameter of the nanotube may be at most 95 % of the average nanotube outer diameter. The average nanotube inner diameter may be at most 90 % of the average nanotube outer diameter. The average nanotube inner diameter may be at most 80 % of the average nanotube outer diameter. The average lateral nanotube spacing may be in the range of 1 nm to 1,000 nm. The average lateral nanotube spacing may also be in the range of 5 nm to 500 nm. The average nanotube lateral spacing may also be in the range of 10 nm to 200 nm. The average nanotube lateral spacing may be 85 nm. The average distance between tubes may be 85 nanometers. The average distance between tubes may be 63 nanometers. An array of nanotubes may comprise at least 1 nanotubes/micrometer² (nanotubes/µm²), at least 10 nanotubes/µm², at least 100 nanotubes/µm², or at least 1,000 nanotubes/µm².

The method comprises reducing the oxygen level at and/or close to the substrate surface below a detection limit of an energy dispersive X-ray spectrometer, wherein the reducing the amount of oxide layer is carried out before the step of providing at least one substrate.

The method may further comprise testing the thereby prepared article to determine re-endothelialization potential of the article. The test may comprise determining number and/or morphology of primary human aortic endothelial cells migrated to, spread, or proliferated on the surface of the article in vitro, and thereby obtaining a test result for re-endothelialization potential of the article.

The method may further comprise testing the substrate prior to the step of the providing at least one substrate, wherein the testing may be done to determine re-endothelialization potential of the substrate. The test may comprise determining number and/or morphology of primary human aortic endothelial cells migrated to, spread, or proliferated on the surface the substrate in vitro, and thereby obtaining a test result for re-endothelialization potential of the substrate.

The method may further comprise comparing the test result for the re-endothelialization potential of the article with the test result for the re-endothelialization potential of the substrate, and deciding whether to use the thereby formed article in manufacturing of a medical device.

The method may further comprise comparing the test result for the re-endothelialization potential of the article with the test result for the re-endothelialization potential of the substrate, and using the thereby formed article in manufacturing of a medical device if number of migrated primary human aortic endothelial cells for the article is higher than that for the substrate.

The method may further comprise comparing the test result for the re-endothelialization potential of the article with the test result for the re-endothelialization potential of the substrate, and using the thereby formed article in manufacturing of a medical device if number of migrated primary human aortic endothelial cells for the article is at least 10% higher than that for the substrate.

The method may further comprise comparing the test result for the re-endothelialization potential of the article with the test result for the re-endothelialization potential of the substrate, and using the thereby formed article in manufacturing of a medical device if number of migrated primary human aortic endothelial cells for the article is at least 50% higher than that for the substrate.

The method may further comprise comparing the test result for the re-endothelialization potential of the article with the test result for the re-endothelialization potential of the substrate, and using the thereby formed article in manufacturing of a medical device if number of migrated primary human aortic endothelial cells for the article is at least 100% higher than that for the substrate.

The method may comprise testing the thereby formed article to determine number and/or morphology of human aortic smooth muscle cells migrated to, spread, or proliferated on the surface of the article in vitro, and thereby obtaining a test result for restenosis reduction potential of the article.

The method may also comprise testing the substrate prior to the step of the providing at least one substrate, wherein the testing comprises determining number and/or morphology of primary human aortic smooth muscle cells migrated to, spread, or proliferated on the surface of the substrate in vitro, and thereby obtaining a test result for restenosis reduction potential of the substrate.

The method may also comprise comparing the test result for the restenosis reduction potential of the article with the test result for the restenosis reduction potential of the substrate, and deciding whether to use the thereby formed article in manufacturing of a medical device.

The method may also comprise comparing the test result for the restenosis reduction potential of the article with the test result for the restenosis reduction potential of the substrate, and using the thereby formed article in manufacturing of a medical device if number of migrated human aortic smooth muscle cells for the article is lower than that for the substrate.

The method comprises comparing the test result for the restenosis reduction potential of the article with the test result for the restenosis reduction potential of the substrate, and using the thereby formed article in manufacturing of a medical device if number of migrated human aortic smooth muscle cells for the article is at least 10% lower than that for the substrate.

The method may also comprise comparing the test result for the restenosis reduction potential of the article with the test result for the restenosis reduction potential of the substrate, and using the thereby formed article in manufacturing of a medical device if number of migrated human aortic smooth muscle cells for the article is at least 50% lower than that for the substrate.

In this method, the step of applying electrical energy between the at least one anode and the at least one cathode may comprise applying a substantially constant voltage. The substantially constant voltage may be in the range of 10 volt to 100 volts. The substantially constant voltage may be about 85 volts. The substantially constant voltage may be about 70 volts.

In this method, the step of applying electrical energy between the at least one anode and the at least one cathode may comprise applying a substantially constant current. The substantially constant current may be in the range of 1 femtoampere to 100 kiloamperes.

In this method, the period may be in the range of 0.1 minute to 1,000 minutes. The period may be in the range of 1 minute to 100 minutes. The period may be about 4 minutes.

In this method, the electrolyte solution may comprise compounds of ammonium fluoride, ethylene glycol, and water.

The method may further comprise maintaining the electrolyte solution at a substantially constant temperature. The substantially constant temperature may be above a freezing point of the electrolyte solution and below a boiling point of the electrolyte solution. The substantially constant temperature may be in the range of 10 °C to 50 °C.

### Examples 1 - 10. Coating Nitinol Surfaces with Nickel-Titanium-Oxide Coatings

In these examples, Nitinol foils ("bare Nitinol foils") were used as the substrates. These foils were coated with coatings comprising a nickel-titanium-oxide compound by using the electrolysis method disclosed above. The details of these examples are as follows.

The bare Nitinol foils were purchased from two different companies: (a) Nitinol Devices & Components, Inc. (NDC, Fremont, CA) provided an about 0.37 mm thick nitinol foil that comprised about 55.85 weight % nickel, about 44.15 weight % titanium and labeled by manufacturer as "light oxide surface" (catalog number 11871), and (b) Alfa Aesar (Ward Hill, MA) provided an about 0.38 mm thick nitinol foil that comprised about 55.75 weight % nickel, about 44.25 weight % titanium and labeled by manufacturer as "superelastic, pickled surface" (catalog number 44954). Surfaces of the bare Nitinol foils before the electrolysis were examined by using a Scanning Electron Microscope (SEM), manufactured by Carl Zeiss Microscopy, LLC (Peabody, MA) with a model name Ultra 55 FE-SEM. The bare Nitinol foils, i.e. the uncoated foils before the electrolysis, had rather smooth surfaces with no distinctive topographical features, as shown in **FIG.2****.**

Each Nitinol foil was cut into a sample of an about 1 centimeter (cm) by about 1 cm piece and cleaned sequentially using an ultrasonicator with dilute micro-90 solution (International Products Corporation, Burlington Township, NJ), acetone and ethanol. The cleaned foil was dried in nitrogen and placed in an anodization setup comprising a Teflon container. The bare Nitinol foil was connected to an electrode to form the working electrode (i.e. anode). A platinum foil was connected to an electrode to form the counter electrode (i.e. cathode). The platinum foil, purchased from Alfa Aesar, was about 0.1 millimeter (mm) thick, about 1.5 cm wide and about 3 long, and was about 99.99 weight % pure. The distance between the anode and the cathode was about 7 cm. The Teflon container was filled with an electrolyte solution, which submerged at least a portion of the anode and at least a portion of the cathode into the electrolyte solution. The electrolyte solution comprised ammonium fluoride (NH₄F), ethylene glycol, and water. NH₄F and ethylene glycol were purchased from Sigma-Aldrich Co. (St. Louis, MO). The water was purchased from EMD Millipore (Billerica, MA). The anodization (i.e. electrolysis) was carried out at a substantially constant voltage for a period of time. The concentration of NH₄F in the electrolyte solution, the voltage, and/or anodization period were varied to prepare articles under different electrolysis conditions. After the electrolysis was completed, the substrates were rinsed copiously with Millipore water and ethanol. Articles, each of which is comprising the bare Nitinol foil (i.e. the substrate) and the coating comprising titanium, nickel and oxygen, were thereby prepared.

In Example 1, a coating comprising nanotubes were obtained with the nitinol foils purchased from Alfa Aesar under the following electrolysis conditions: the electrolyte solution comprised about 1.48 grams (g) of NH₄F, about 490 milliliters (ml) of ethylene glycol, and about 8.35 ml of water; and the anodization voltage and duration were about 85 volts (V) and about 4 minutes (min) respectively. The SEM image of this coating is shown in **FIG.3****.** The coating was rather uniform and continuous, **FIG.3A** and comprised nanotubes, **FIG.3B****.** The average outer diameter of these nanotubes was about 110 nm ± 40 nm (varying in the range of about 130 nm to about 70 nm). The average inner diameter of these nanotubes was about 90.6 nm ± 11.1 nm. The average length of these nanotubes was about 753 nm ± 113 nm. The lateral spacing between the nanotubes was about 86 nm ± 36 nm (varying in the range of about 30 nm to about 170 nm). These nanotubes comprised an array of 50 ±1 nanotubes per micrometer square (nanotubes/µm²).

In Example 2, a coating comprising nanotubular structures were obtained with the Nitinol foils purchased from Alfa Aesar under the following electrolysis conditions: the electrolyte solution comprised about 1.48 grams (g) of NH₄F, about 490 milliliters (ml) of ethylene glycol, and about 8.35 ml of water; and the anodization voltage and duration were about 70 V and about 4 min respectively. The coating comprised nanotubes, with an average outer diameter of about 90 nm ± 15 nm (varying in the range of about 69 nm to about 110 nm), as shown in **FIG.4****.** The average inner diameter of these nanotubes was about 72.3 nm ± 15.3 nm. The lateral spacing between the nanotubes was about 63 nm ± 40 nm (varying in the range of about 11 nm to about 150 nm). These nanotubes comprised an array of 1,600 ± 80 nanotubes/µm².

In Examples 3-10, eight articles comprising porous nickel-titanium-oxide coatings were obtained under variety of electrolysis conditions, as shown in **FIGs.5-12****.** Some of these coatings had average pore sizes in the range of about 20 to about 100 nm. For example, in Figure 8, the coating has rather uniform pores in the range of 60 nm to 100 nm.

Surfaces of the Nitinol foil purchased from Alfa Aesar (i.e. control or the substrate) and the article prepared in Example 1 were analyzed by an energy dispersive X-ray spectrometer (EDS) (Oxford Instruments, X-Max, 80 mm² detector area). The EDS of the bare Nitinol foil is shown in **FIG.13****.** This analysis determined that the Nitinol foil comprised about 58.89 weight % nickel and about 41.11 weight % titanium. The surface and/or layer close to the surface of this foil was substantially free of oxygen. That is, the oxygen level of the bare Nitinol foil purchased from Alfa Aesar was below the detection limit of the EDS. The EDS of the article prepared in Example 1, shown in **FIG.14****,** indicated that the nanotubular structures shown in **FIG.3** comprised about 36.45 weight % nickel, about 32.14 weight % titanium, and about 31.41 weight % oxygen. The calculations based on this elemental analysis showed that these nanotubular structures may comprise NiO and TiO₂.

### Example 11. Leaching of Nickel Ions from Articles and Substrates

In this example, leaching of nickel ions from the article prepared in Example 1 and the Nitinol foil purchased from Alfa Aesar was determined. About 1 cm x about 1 cm samples of the article and the substrate were submerged in tissue culture plates containing about 1 ml of a Phosphorous Buffer Saline (PBS) solution in a standard 24-well tissue culture plate and placed in an incubator under standard tissue culture conditions (about 37°C, about 5% CO₂, and about 95% humidity). About 1 ml of PBS samples were retrieved at specific time points and replaced with fresh PBS solution. These PBS samples were then tested using the Nickel Assay Kit (Sigma-Aldrich, catalog number MAK027) following the manufacturer's instructions.

The results shown in **FIG.15** indicated that the articles comprising nanotubular coatings had higher nickel release rate than that of the bare Nitinol foil (i.e. control). The articles comprising nanotubular coatings had an average daily release rate of about 37.6 ± 8.2 nanograms (ng) per sample while the control Nitinol substrates had an average daily release rate of 8.8 ± 5.0 ng per sample.

The cumulative nickel released from the article comprising the nanotubular coatings reached as high as about 2.5 micrograms (µg) at the end of about 45 days, while the cumulative nickel released from the bare Nitinol foil was lower than about 0.8 µg for the same period. However, these release rates were much lower than the intravenous nickel contamination limit of about 1,575 µg after about 45 days (or about 35 µg/day) for a about 70 kg man, as shown in **FIG.16****.** For the intravenous nickel contamination limits, see Sunderman, F. J. "Potential Toxicity from Nickel Contamination of Intravenous Fluids" Ann. Clin. Lab. Sci., 1983, 13, 1-4. The entire content of this publication is incorporated herein by reference.

These results indicated that a medical article comprising a Nitinol substrate and a nanotubular coating comprising a compound of nickel, titanium and oxygen may safely be used in vivo, for example, as an implant in a cardiovascular system of a human.

### Example 12. Migration of Endothelial Cells on Surfaces of the Articles Comprising Nickel-Titanium-Oxide Coatings.

In this example, re-endothelialization potential of the article comprising a coating of a compound of nickel, titanium and oxygen was determined by quantifying the migration of primary human aortic endothelial cells (HAEC) on a surface of the coating.

Re-endothelialization is one of the key factors in the prevention of restenosis and thrombosis since the process of stent deployment causes denudation of the endothelial layer as disclosed, for example, in publications: Steffel et al. "Biological Effects of Drug-Eluting Stents in the Coronary Circulation" Herz 2007, 32, 268-273; Peng et al. "The Effect of TiO2 Nanotubes on Endothelial Function and Smooth Muscle Proliferation" Biomaterials 2009, 30, 1268-1272; Peng "Whole Genome Expression Analysis Reveals Differential Effects of TiO2 Nanotubes on Vascular Cells" Nano Lett. 2010, 10, 143-148; and Chan et al "In Vivo Prevention of Arterial Restenosis with Paclitaxel-Encapsulated Targeted Lipid-Polymeric Nanoparticles" Proc. Natl. Acad. Sci. U. S. A. 2011, 108, 19347-19352. The entire content of each of these publications are incorporated herein by reference.

In this example, the article prepared in the same manner described in Example 1 was used. The migration potential of HAEC on the surface of the bare Nitinol foil (i.e. control or the substrate) purchased from Alfa Aesar was also similarly investigated. HAEC used in this Example were manufactured by Lonza (USA). Cells were maintained and cultured under manufacturer's instructions and all experiments were conducted with cells between passage numbers 3 and 7.

The endothelial cell migration potential was determined by using a method similar to that disclosed in a publication by Sprague et al. "Human Aortic Endothelial Cell Migration onto Stent Surfaces under Static and Flow Conditions" J. Vasc. Interv. Radiol., 1997, volume 8, pages 83-92. The entire content of this publication is incorporated herein by reference. In this method, first, HAEC were seeded onto a rat tail collagen gel with a concentration of about 4mg/ml (Fisher, USA) in a 12-well tissue culture plate, and cultured to confluency, as schematically shown in **FIG.17A****.** Then, a sample of the bare Nitinol foil, and a sample of the article comprising the coating, each with a size of about 1 cm x about 1 cm, were carefully placed on the surface of the cultured cells and the gel, as shown in **FIG.17B****.** When the article was placed, the uncoated surface of the article faced the surface of the cells and the gel. That is, the coating comprising nanotubular structures faced up, i.e. faced in the opposite direction, when the article was placed. After about 4 days of culturing, the samples were carefully removed, as shown in **FIG.17C****,** and transferred to fresh 24-well tissue culture plates. The number of endothelial cells on each sample surface was quantified using the CyQuant assay (Molecular Probes, USA) according to the manufacturer's instructions. The number of HAEC on the article comprising the nickel-titanium-oxide coating, 32,594 ± 4,197 cells was significantly higher than that on the substrate, bare Nitinol foil (i.e. control), 10,470 ± 1,884 cells as shown in **FIG.18****.** (N = 5, paired t-test, p < 0.001.)

These results implied that since the endothelial cell migration is the key part to wound healing, a modification of the surface of the stent by a porous nickel-titanium oxide coating would aid the migration of HAEC onto the stent surface and would provide better and/or quicker healing of the endothelium as compared to the bare metal stents. The recovery of the endothelium would also lead to the reduction in the migration of the HASMC into the intima and the proliferation of these cells, ultimately improving the overall viability of the vessel.

### Example 13. Spreading and Proliferation of Endothelial Cells on Surfaces of the Articles Comprising Nickel-Titanium-Oxide Coatings.

In this example, endothelial cell spreading and proliferation potential of the articles comprising a coating of a compound of nickel, titanium and oxygen was determined. Such test may be another type of test to determine re-endothelialization potential of the article.

In this example, the article prepared in the same manner described in Example 1 was used. The cell spreading and proliferation potential of HAEC on the surface of the bare Nitinol foil (i.e. control or the substrate) purchased from Alfa Aesar was also similarly investigated. HAEC used in this example were manufactured by Lonza (USA). Cells were maintained and cultured under manufacturer's instructions and all experiments were conducted with cells between passage numbers 3 and 7.

Samples were sterilized with ethanol and rinsed twice with sterile PBS in 24-well tissue culture plates (VWR, USA) under sterile conditions. HAEC were seeded at a cell density of 10,000 cells/well and cultured under manufacturer's instructions. At about day 1, about day 4 and about day 7, the samples were rinsed in sterile PBS and transferred into fresh 24-well plates. The cells were fixed with about 3.7% paraformaldehyde and blocked using a solution of about 2.5% Bovine Serum Albumin, about 0.1% triton-X and PBS. They were then actin-stained using FITC-conjugated Phalloidin and nuclei-stained using DAPI, following manufacturer's instructions (Millipore, USA). These samples were then imaged under a Nikon C1 si spectral confocal microscope. For exemplary images, see **FIG.19A and FIG.19B****.** Using ImageJ, we quantified cell spreading **(****FIG. 20A****)** on these substrates by measuring the average surface area per cell by the actin staining and normalizing it to the control. The cell number was also determined with ImageJ and the results can be seen in **FIG. 20B****.**

There was a morphological difference between HAEC cultured on the bare Nitinol foil and the article comprising nanotubular coating, as shown in **FIG. 20****.** HAEC cultured on the article comprising nanotubular coating appeared to be individually larger and more spread out than those cultured on the bare Nitinol foil. HAEC on the article also displayed a more elongated and extended morphology. These results indicated that endothelial cell spreading and proliferation potential of the article comprising nanotubular coating was better than that of the bare Nitinol foil.

The relative cell surface area of HAEC cultured on the article was about two and a half times larger than that of the substrate, as shown in **FIG.20A****.** Thus, the endothelial cells spread and proliferated more successfully on the surface of the article than on the surface of the substrate.

As shown in **FIG.20B****,** the number of HAEC on nanotubular coated Nitinol was similar over the course of about 7 days in the culture while the number of HAEC on the control increased over the course of about 7 days in the culture. This showed that proliferation of HAEC was not increased on the article comprising nanotubular coating. This may be because the spreading and proliferation of HAEC reached confluency on the article comprising the nanotubes within about 1 day.

Above results implied that a modification of the surface of the stent by a porous nickel-titanium oxide coating would aid the spread and proliferation of HAEC on the stent surface, after migration of these cells, and would provide better and/or quicker healing of the endothelium as compared to the bare metal stents.

### Example 14. Reduction of Restenosis by the Articles Comprising Nickel-Titanium-Oxide Coatings.

In this example, restenosis reduction potential of the article comprising a coating of a compound of nickel, titanium and oxygen was determined by quantifying the migration, cell spreading and proliferation of Human Aortic Smooth Muscle Cells (HASMC) on the surface of the coating. The article used in this example was prepared in the same manner described in Example 1. The restenosis reduction potential of the substrate, i.e. bare Nitinol foil purchased from Alfa Aesar (i.e. control) was also similarly investigated.

HASMC can migrate through the vessel walls, begin proliferating excessively and release Extracellular Matrix (ECM) proteins such as collagen, resulting in restenosis, as explained in a publication by Rajagopal "Coronary Restenosis: A Review of Mechanisms and Management" Am. J. Med. 2003, volume 115, pages 547-553. The entire content of this publication is incorporated herein by reference. Vascular smooth muscle cell proliferation has been observed in neointimal hyperplasia, as disclosed in the Rajagopal publication, and also in a publication by Lüscher et al. "Drug-Eluting Stent and Coronary Thrombosis: Biological Mechanisms and Clinical Implications" Circulation 2007, 115, 1051-1058.The entire content of each of these publications is incorporated herein by reference.

HASMC used in this example were manufactured by Lonza (USA). Cells were maintained and cultured under manufacturer's instructions and all experiments were conducted with cells between passage numbers 3 and 7.

The migration, spreading and proliferation of HASMC on the article and the substrate (i.e. control) were determined in the same manner disclosed in Example 11 and 12 for the HAEC migration, cell spreading and proliferation. Briefly, the HASMC were seeded at a density of about 10,000 cells/well in 24-well tissue culture plates. At days of about 1, about 4 and about 7, the samples were rinsed with PBS, transferred to fresh 24-well tissue culture plates, and actin- and nuclei-stained with DAPI and FITC-Phalloidin respectively. The cells were imaged and counted in ImageJ. The results are shown in **FIGs. 21-22****.**

These results indicated that the number of HASMC on the article comprising the nanotubular coating was lower than that on the bare Nitinol foil (i.e. control), as shown in **FIG.22****.** (For each bar on the figure: N = 5, paired t-test, p < 0.01.) At about 7^{th} day, the number of HASMC migrated on the surface of the bare Nitinol foil was significantly higher than that on the surface of the article comprising nanotubular coating. Furthermore, at about 7^{th} day, the HASMC on the bare Nitinol foil have begun to reach confluency, as seen on the images in **FIG.21B****.** In the confluent cell clusters, the HASMC also began to align on the surface of the bare Nitinol foil. In contrast, the HASMC on the article comprising the nanotubular coating are less confluent and not as aligned, as shown in **FIG.21A****.** These results implied that, for example, for a stent comprising a porous nickel-titanium-oxide coating, the HASMC that have migrated to the lumen and come into contact with the porous coating will exhibit less proliferation. The stents comprising such porous coatings may prevent or reduce vascular smooth muscle cell proliferation, and thereby provide better medical solutions for cardiovascular treatments as compared to bare metal stents.

### Example 15. Effects of Porous Coatings Comprising Nickel-Titanium-Oxides on Relative mRNA Expression Levels

Another major component of restenosis is the release of ECM proteins by HASMC that are activated at the lesion site. In this example, using quantitative Polymerase Chain Reaction (PCR) method, effects of the porous coatings on the relative mRNA expression levels of two major ECM proteins, collagen I (Col1) and collagen III (Col3) were investigated.

Relative mRNA expression levels of matrix metalloproteinase 2 (MMP2) have been shown to be related to the migration of HASMC, as disclosed in a publication by Pauly et al. " Migration of Cultured Vascular Smooth Muscle Cells through a Basement Membrane Barrier Requires Type IV Collagenase Activity and Is Inhibited by Cellular Differentiation" Circ. Res. 1994, volume 75, pages 41-54. The entire content of this publication is incorporated herein by reference. Therefore, effects of the porous coatings on the relative mRNA expression levels of MMP2 were also investigated in this example.

The article comprising a nanotubular coating used in this example was prepared in the same manner described in Example 1. Bare Nitinol foil purchased from Alfa Aesar (i.e. control) was also similarly investigated. HASMC were seeded on about 1 cm by about 1cm samples of the Nitinol foil and the article comprising the nanotubular coating in 24-well tissue culture plates at a seeding density of about 30,000 cells/well. After about 48 hours, the samples were transferred into fresh 24-well tissue culture plates and about 0.5% EDTA was used to lift the cells from each substrate. After about 6 minutes of exposure to EDTA, the cell suspension was transferred to an Eppendorf tube and centrifuged before aspirating the solution. mRNA isolation was performed on the cells using the Qiagen RNeasy Mini Kit (Qiagen, USA) and TRIzol Reagent (Ambion) following manufacturer's instructions. mRNA concentration and purity were determined using the Nano Drop ND-1000 Spectrophotometer (Thermo Scientific, USA). iScript cDNA Synthesis Kit (Bio-Rad, USA) was used to synthesize cDNA. Then, forward and reverse primers and Fast SYBR Green Master Mix (Applied Biosystems, USA) were used to amplify the cDNA of interest in an Applied Biosystems Viia7 real-time polymerase chain reaction system. Biological triplicates were performed for each substrate and normalized to GAPDH expression.

As shown in **FIG.23****,** using GAPDH as the reference gene, the mRNA expression levels of collagen I were significantly downregulated on the article comprising the nanotubular coating as compared to bare Nitinol foil (i.e. control). Collagen is a major component of the neointima, as disclosed in the Rajagopal publication and in a publication by Nagler et al. "Inhibition of Collagen Synthesis, Smooth Muscle Cell Proliferation, and Injury-Induced Intimal Hyperplasia by Halofuginone" Arterioscler. Thromb. Vasc. Biol. 1997, volume 17, pages 194-202. The entire content of each of these publications is incorporated herein by reference. Therefore, these results indicated that the collagen may be downregulated by use of an article comprising a porous nickel-titanium-oxide coating. Thus, these results also implied that using, for example, a stent comprising a porous nickel-titanium-oxide coating may reduce the restenosis by directly affecting the formation of the neointima by downregulating the mRNA expression levels of collagen I in HASMC that are in contact with the surface of the medical article.

Furthermore, HASMC are known to produce MMP-2 to degrade the vessel basement membrane in order to migrate into the intima, as disclosed in a publication by Chen et al. "PDGF-D Contributes to Neointimal Hyperplasia in Rat Model of Vessel Injury" Biochem. Biophys. Res. Commun. 2005, volume 329, pages 976-983. The entire content of this publication is incorporated herein by reference. Thus, the downregulation in the mRNA expression levels of MMP-2 in HASMC may imply the reduction in the migration rates of the HASMC on the surface. If the HASMC in contact with a porous surface secrete less MMP-2, the article (e.g. a stent) comprising such porous nickel-titanium-oxide surfaces may reduce the migration of other HASMC traveling into the intima, thereby reducing the restenosis.

### Example 16. Effects of Varying Nanotube Diameter on HASMC Adhesion and Growth

Effects of varying nanotube diameter on HASMC adhesion and growth were disclosed in this example. Coatings comprising nanotubes with average diameters of about 110 nm, about 90 nm, or about 70nm were formed on Nitinol substrates. Bare (i.e. uncoated) Nitinol foil prepared according to the method disclosed above was used as a control. The Nitinol foils were obtained from Alfa Aesar (MA, USA). Each substrate was about 1 cm x about 1 cm in size. HASMC (Lonza, USA) in the 4^{th} passage of cell culture were used.

Each Nitinol substrate was sterilized with ethanol and rinsed twice with sterile PBS in 24-well tissue culture plates (VWR, U.S.A.) under sterile conditions. HASMC were seeded at a cell density of about 10,000 cells/well and cultured under manufacturer's instructions. At about 1 days and about 7 days of culture, the Nitinol substrates were rinsed in sterile PBS and transferred into fresh 24-well plates. The cells were fixed with about 3.7 % paraformaldehyde and blocked using a solution of about 2.5 % bovine serum albumin, about 0.1 % Triton-X and PBS. They were then actin-stained using FITC-conjugated Phalloidin and nuclei-stained using DAPI, following manufacturer's instructions (Millipore, USA). These samples were then imaged under a Nikon C1 si spectral confocal microscope **(****FIG. 24****).** The average cell number was obtained by counting with ImageJ and is shown in **FIG. 25** and **FIG. 26****.**

As seen in **FIG. 24****,** the surfaces of the control Nitinol substrates have started to become confluent with HASMC. The HASMC on the control substrates demonstrated greater cell alignment than any of the other experimental groups, although major differences in cell alignment were not observed between the articles comprising nanotubes with average inner diameters of about 110 nm ("110"), about 90 nm ("90") and about 70 nm ("70"). In **FIG. 25****,** at day 1, there is a significant increase in the number of HASMC on the control Nitinol substrates as compared to the 110 and 90 groups (N=5, p<0.05, one-tail t-test). We can also observe a trend of HASMC numbers increasing as the nanotube diameter decreases. Furthermore, there is a significant increase in the number of HASMC in the 70 group as compared to the 110 group (N=5, p<0.05, one-tail t-test). Therefore, HASMC may demonstrate weaker cellular adhesion to nanotubes-coated Nitinol substrates as the diameter of the nanotubes increases. Cellular adhesion is one of the earlier forms of interaction between cells and implants, and reducing HASMC adhesion to the stent is desirable for reducing restenosis.

Also in **FIG. 25****,** at day 7, we observe a significant increase in the number of HASMC on the control Nitinol substrates as compared to the 110, 90 and 70 groups (N=5, p<0.05, one-tail t-test). We further analyzed the data by looking at the difference in the HASMC number between Day 7 and Day 1 on each type of substrates **(****FIG.** 26). By taking into account the number of HASMC that adhered at Day 1, we can analyze the growth of the HASMC that were on each type of substrate. As seen in **FIG. 26****,** only the 110 and 70 groups demonstrated statistical significance in decreasing the growth of HASMC as compared to the control Nitinol substrates. Therefore, these 2 diameters may be more efficient in decreasing restenosis in stents.

We also investigated the effects of varying nanotube diameters on the adhesion of HAEC, since HAEC adhesion is necessary for the healing of the endothelium in the context of stenting. The same experimental groups and substrates were used for this experiment. HAEC (Lonza (USA)) in the 4^{th} passage of cell culture were used. HAEC were seeded at a density of 12,000 cells/well and cultured under manufacturer's conditions. At day 1, cells were fixed, nuclei-stained, imaged under confocal microscopy and counted in ImageJ **(****FIG. 27****).** As can be observed in **FIG. 27****,** there is no statistically significant difference in the number of HAEC that adhered to the substrates. This shows that our coating does not affect the cellular adhesion of HAEC as compared to the control. Taken together with the data from **FIG. 24** to **FIG. 27****,** we have demonstrated for the first time that by varying the nanotube diameters on our Nitinol-based nanotubular coatings, we can modulate HASMC adhesion without compromising on HAEC adhesion.

Above Examples indicated that variety of stents comprising porous nickel-titanium-oxide coatings may be prepared. Such article may be prepared by using an electrolysis method. The porous structure of these coatings may be affected by varying the anodization conditions, such as electrolysis voltage, electrolysis period, and electrolyte composition. Well-defined porous coatings, such as coatings comprising tubular structures may also be prepared by varying the anodization conditions. The nickel release rates from these porous coatings were well below the biological safety limits, indicating that the articles with such porous coatings may safely be used in manufacturing of medical devices, such as stents. These Examples also show that the restenosis may be reduced or prevented by reducing the proliferation, ECM production and migration of HASMC. Furthermore, since we have demonstrated the "pro-healing" properties of our nanotubular coating via the significant increase in cell spreading and migration of HAEC onto our nanotubes-coated Nitinol substrates, a key factor in the recovery of the endothelium post-stenting.

In this disclosure, the indefinite article "a" and phrases "one or more" and "at least one" are synonymous and mean "at least one".

Relational terms such as "first" and "second" and the like may be used solely to distinguish one entity or action from another, without necessarily requiring or implying any actual relationship or order between them. The terms "comprises," "comprising," and any other variation thereof when used in connection with a list of elements in the specification or claims are intended to indicate that the list is not exclusive and that other elements may be included. Similarly, an element preceded by an "a" or an "an" does not, without further constraints, preclude the existence of additional elements of the identical type.

## Claims

1. A stent comprising:
a substrate comprising at least one surface, and
a coating comprising a compound of nickel, titanium and oxygen,
wherein the coating is formed on the at least one surface of the substrate, and wherein the coating is a porous coating comprising an array of nanotubes, wherein the nanotubes have an average inner diameter of 70 nm or 110 nm.

2. The stent of any of the preceding claims, wherein the substrate comprises a metal.

3. The stent of any of the preceding claims, wherein the substrate comprises a stainless steel, an alloy of cobalt and chromium, an alloy of nickel and titanium, or mixtures thereof.

4. The stent of any of the preceding claims, wherein the substrate comprises an alloy of nickel and titanium.

5. The stent of any of the preceding claims, wherein the substrate comprises nitinol.

6. The stent of any of the preceding claims, wherein the porous coating comprises a nanotube array of at least 1 nanotube/micrometer².

7. The stent of any of the preceding claims, wherein the porous coating comprises a nanotube array of at least 10 nanotubes/micrometer².

8. The stent of any of the preceding claims, wherein the porous coating comprises a nanotube array of at least 100 nanotubes/micrometer².

## Patentansprüche

1. Stent, aufweisend:
ein Substrat, das mindestens eine Oberfläche aufweist; und
eine Beschichtung, die eine Verbindung aus Nickel, Titan und Sauerstoff aufweist,
wobei die Beschichtung auf der mindestens einen Oberfläche des Substrats ausgebildet ist, und wobei die Beschichtung eine poröse Beschichtung ist, die eine Anordnung von Nanoröhren aufweist, wobei die Nanoröhren einen durchschnittlichen Innendurchmesser von 70 nm oder 110 nm haben.

2. Stent nach einem der vorhergehenden Ansprüche, bei dem das Substrat ein Metall aufweist.

3. Stent nach einem der vorhergehenden Ansprüche, bei dem das Substrat einen rostfreien Stahl, eine Legierung aus Kobalt und Chrom, eine Legierung aus Nickel und Titan, oder Mischungen davon aufweist.

4. Stent nach einem der vorhergehenden Ansprüche, bei dem das Substrat eine Legierung aus Nickel und Titan aufweist.

5. Stent nach einem der vorhergehenden Ansprüche, bei dem das Substrat Nitinol aufweist.

6. Stent nach einem der vorhergehenden Ansprüche, bei dem die poröse Beschichtung eine Nanoröhrenanordnung von mindestens 1 Nanoröhre/Mikrometer² aufweist.

7. Stent nach einem der vorhergehenden Ansprüche, bei dem die poröse Beschichtung eine Nanoröhrenanordnung von mindestens 10 Nanoröhren/Mikrometer² aufweist.

8. Stent nach einem der vorhergehenden Ansprüche, bei dem die poröse Beschichtung eine Nanoröhrenanordnung von mindestens 100 Nanoröhren/Mikrometer² aufweist.

## Revendications

1. Endoprothèse comprenant :
un substrat comprenant au moins une surface ; et
un revêtement comprenant un composé de nickel, de titane et d'oxygène,
le revêtement étant formé sur l'au moins une surface du substrat, et le revêtement étant un revêtement poreux comprenant un réseau de nanotubes, les nanotubes ayant un diamètre interne moyen de 70 nm ou 110 nm.

2. Endoprothèse selon l'une quelconque des revendications précédentes, dans lequel le substrat comprend un métal.

3. Endoprothèse selon l'une quelconque des revendications précédentes, dans lequel le substrat comprend un acier inoxydable, un alliage de cobalt et de chrome, un alliage de nickel et de titane, ou des mélanges de ceux-ci.

4. Endoprothèse selon l'une quelconque des revendications précédentes, dans lequel le substrat comprend un alliage de nickel et de titane.

5. Endoprothèse selon l'une quelconque des revendications précédentes, dans lequel le substrat comprend du nitinol.

6. Endoprothèse selon l'une quelconque des revendications précédentes, dans lequel le revêtement poreux comprend un réseau de nanotubes d'au moins 1 nanotube/micromètre².

7. Endoprothèse selon l'une quelconque des revendications précédentes, dans lequel le revêtement poreux comprend un réseau de nanotubes d'au moins 10 nanotubes/micromètre².

8. Endoprothèse selon l'une quelconque des revendications précédentes, dans lequel le revêtement poreux comprend un réseau de nanotubes d'au moins 100 nanotubes/micromètre².
